# EUROPÄISCHE PATENTSCHRIFT

(11) **EP 2 352 720 B1**
(45) Veröffentlichungstag und Bekanntmachung des Hinweises auf die Patenterteilung: **20.12.2017**
(21) Anmeldenummer: 09751855.9
(22) Anmeldetag: 29.10.2009
(51) Int. Cl.: C07C 231/06, C07C 231/14, C07C 253/00, C07C 233/09, C07C 255/16

(54) **VERFAHREN ZUR HERSTELLUNG VON EINEM CARBONSÄUREAMID AUS EINER CARBONYLVERBINDUNG UND BLAUSÄURE**
METHOD FOR PRODUCING A CARBOXYLIC ACID AMIDE FROM A CARBONYL COMPOUND AND HYDROCYANIC ACID
PROCÉDÉ DE PRÉPARATION D'UN AMIDE D'ACIDE CARBOXYLIQUE À PARTIR D'UN COMPOSÉ CARBONYLE ET D'ACIDE CYANHYDRIQUE

(30) Priorität: 01.12.2008 DE 102008044218
(43) Veröffentlichungstag der Anmeldung: 10.08.2011
(73) Patentinhaber: Evonik Röhm GmbH, 64293 Darmstadt (DE)
(72) Erfinder: MAY, Alexander, 64342 Seeheim (DE); KÖSTNER, Martin, 64295 Darmstadt (DE); BECKER, Jörg, 64295 Darmstadt (DE); SCHALLENBERG, Jörg, 63791 Karlstein (DE); SIEGERT, Hermann, 64342 Seeheim-Jugenheim (DE); VOGEL, Bernd, 65195 Wiesbaden (DE)
(86) Internationale Anmeldenummer: PCT/EP2009/064279
(87) Internationale Veröffentlichungsnummer: WO 2010/063520

(56) Entgegenhaltungen:
- EP-A2- 0 407 811
- DE-A1-102006 055 430
- JP-A- H06 172 283
- US-A- 4 018 829
- GERHARTZ, W. ET AL.: "10. DERIVATIVES 10.1 ACETONE CYANOHYDRIN", ULLMANN'S ENCYCLOPEDIA OF INDUSTRIAL CHEMISTRY, vol. A1, 1985, pages 91-92, XP003032982, ISBN: 978-3-527-20101-3

## Beschreibung

Die vorliegende Erfindung betrifft ein Verfahren zur Herstellung von einem Carbonsäureamid aus einer Carbonylverbindung und Blausäure. Des Weiteren betrifft die vorliegende Erfindung ein Verfahren zur Herstellung von Methylmethacrylat. Die Herstellung von Carbonsäureamiden durch die Hydrolyse von Carbonsäurenitrilen in Gegenwart eines Mangandioxid umfassenden Katalysators ist seit langem Stand der Technik. Carbonsäureamide werden vielfach in der Technik als Zwischenprodukt benötigt. So kann beispielsweise α-Hydroxyisobuttersäureamid zur Herstellung von Methacrylsäure oder Methacrylsäureestern, insbesondere Methylmethacrylat dienen.

In der EP 0407811 wird ein Verfahren zur Herstellung von Methylmethacrylat aus Aceton und Methylformiat oder aus Aceton, Methanol und Kohlenmonoxid als Ausgangsmaterialien beschrieben. Eine Rückgewinnung der eingesetzten Edukte sowie die Verhinderung des Zusetzens der Kolonne durch die Polymerisation von HCN werden nicht beschrieben.

Die US 4018829 beschreibt ein Verfahren zur Herstellung von α-Hydroxyisobutyramid durch Hydratation von Acetoncyanhydrin in Gegenwart von Braunstein als Katalysator.

In der JP H06 172283 wird ebenfalls ein Verfahren zur Herstellung von α-Hydroxyisobuttersäureamid beschrieben. Zur Aufarbeitung wird die α-Hydroxyisobuttersäureamid-Lösung in einen Pyrolysereaktor eingeleitet, um Acetoncyanhydrin zu Aceton und Blausäure zu zersetzen.

Die im Stand der Technik beschriebenen Verfahren erfordern vor der Hydrolyse des Hydroxycarbonsäurenitrils eine destillative Aufreinigung des Reaktionsgemisches.

Ein besonders bevorzugtes Verfahren zur Herstellung von Carbonsäureamiden wird in WO 2008/061822 A1 dargelegt.

Obwohl die Lehre des zuvor dargelegten Dokuments bereits zu einer relativ kostengünstigen Herstellung führt, besteht ein permanentes Bedürfnis das Verfahren weiter zu verbessern. Üblich wird das durch Umsetzung von Blausäure und Carbonylverbindung, insbesondere Aceton erhaltene Cyanhydrin durch Zugabe von Säure stabilisiert. Diese Stabilisierung muss vor der Umsetzung des Cyanhydrins zum Carbonsäureamid aufgehoben werden, wobei üblich eine Destillation erfolgt. In WO 2008/061822 A1 wird dargelegt, dass die nach der Umsetzung von Aceton mit Blausäure erhaltene Mischung zur Hydrolyse eingesetzt werden kann. Allerdings bleibt offen, ob eine Aufreinigung zu erfolgen hat. Im Allgemeinen umfasst diese Aufreinigung eine zweistufige Destillation, wobei in einer ersten Stufe die nicht umgesetzten Edukte abgetrennt werden. In einer zweiten Stufe wird üblich die zur Stabilisierung eingesetzte Säure aus dem Cyanhydrin entfernt. Führt man keine Aufreinigung, beispielsweise Destillation durch, so werden relativ kurze Katalysatorstandzeiten erhalten. Bei Verwendung einer destillierten Reaktionsmischung kann die Standzeit des Katalysators erheblich verbessert werden, wobei hierdurch jedoch die Gesamteffizienz des Verfahrens aufgrund des Energieverbrauchs durch die Destillation verschlechtert wird.

In Anbetracht des Standes der Technik ist es nun Aufgabe der vorliegenden Erfindung Verfahren zur Herstellung von Carbonsäureamiden zur Verfügung zu stellen, die besonders einfach, kostengünstig und mit hoher Ausbeute durchgeführt werden können. Ein besonderes Problem bestand insbesondere darin ein Verfahren zu schaffen, das bei hoher Geschwindigkeit, geringem Energieeinsatz und geringen Ausbeuteverlusten eine besonders lange Lebensdauer des Katalysators und eine lange Standzeit der Anlage zur Herstellung des Carbonsäureamids gewährleistet.

Gelöst werden diese sowie weitere nicht explizit genannte Aufgaben, die jedoch aus den hierin einleitend diskutierten Zusammenhängen ohne weiteres ableitbar oder erschließbar sind, durch ein Verfahren mit allen Merkmalen des Patentanspruchs 1. Zweckmäßige Abwandlungen der erfindungsgemäßen Verfahren werden in Unteransprüchen unter Schutz gestellt.

Gegenstand der vorliegenden Erfindung ist dementsprechend ein Verfahren zur Herstellung von einem Carbonsäureamid aus einer Carbonylverbindung und Blausäure, umfassend die Schritte
A) Umsetzung einer Carbonylverbindung mit Blausäure zur Herstellung eines Hydroxycarbonsäurenitrils,
B) Hydrolyse des in Schritt A) erhaltenen Hydroxycarbonsäurenitrils in Gegenwart eines Mangandioxid umfassenden Katalysators, wobei zur Umsetzung der Carbonylverbindung mit Blausäure gemäß Schritt A) ein molarer Überschuss an Carbonylverbindung in Bezug auf die Blausäure eingesetzt wird und die in Schritt A) erhaltene Reaktionsmischung nicht durch Destillation aufgereinigt wird, bevor die Hydrolyse gemäß Schritt B) durchgeführt wird, welches dadurch gekennzeichnet ist, dass die nach Schritt B) erhaltene Reaktionsmischung durch eine zweistufige Destillation aufgereinigt wird und dass das erhaltene Carbonsäureamid und Wasser von einer Mischung getrennt wird, die Carbonylverbindung und Hydroxycarbonsäurenitril und/oder Blausäure umfasst, und in einem weiteren Destillationsschritt Wasser von erhaltenem Carbonsäureamid getrennt wird, und über den Rücklauf in zur Trennung von Wasser und Blausäure eingesetzte Destille eine Zusammensetzung eingeleitet wird, die die in Schritt A) eingesetzte Carbonylverbindung umfasst, wobei die in den Rücklauf eingeleitete Zusammensetzung einen geringeren Anteil and HCN aufweist, als die über Kopf der Destille entnommene Zusammensetzung, wobei die in Schritt A) eingesetzte Carbonylverbindung Aceton ist. Hierdurch gelingt es überraschend ein besonders energieeffizientes Verfahren zur Verfügung zu stellen, welches lange Katalysatorstandzeiten ermöglicht.

Zugleich lassen sich durch die erfindungsgemäßen Verfahren eine Reihe weiterer Vorteile erzielen. Hierzu gehört unter anderem, dass durch das erfindungsgemäße Verfahren die Standzeit der Anlage zur Herstellung des Carbonsäureamides überraschend stark verlängert werden kann. Hierdurch kann das Verfahren besonders effizient, kostengünstig, bei hoher Geschwindigkeit, mit geringem Energieeinsatz und geringen Ausbeuteverlusten durchgeführt werden.

Das erfindungsgemäße Verfahren ermöglicht die effiziente Herstellung von Carbonsäureamiden. Hierbei wird Aceton als Carbonylverbindung eingesetzt. Aceton kann mit Blausäure (HCN) zu 2-Hydroxy-2-methylpropionitril (Acetoncyanhydrin) umgesetzt werden. Die Carbonylverbindung wird in einem molaren Überschuss, bezogen auf die Blausäure eingesetzt. Das molare Verhältnis von Carbonylverbindung zu Blausäure liegt im Bereich von 2:1 bis 7:1, vorzugsweise im Bereich von 2:1 bis 3:1. Vorzugsweise erfolgt die Umsetzung der Carbonylverbindung mit Blausäure gemäß Schritt A) in Gegenwart einer Base. Hierbei können Anionenaustauscher verwendet werden. Bevorzugt werden Hydroxide oder Oxide eingesetzt werden, die besonders bevorzugt von Erdalkali- oder Alkalimetallen gebildet werden. Hierzu gehören unter anderem Ca(OH)₂ und Mg(OH)₂, MgO, CaO, NaOH, KOH, LiOH oder Li₂O. Ganz besonders bevorzugt wird hierbei LiOH oder Li₂O eingesetzt. Vorzugsweise werden der Reaktionsmischung zur Umsetzung der Carbonylverbindung mit Blausäure 0,001 bis 10 Gew.-%, besonders bevorzugt 0,01 Gew.-% bis 2 Gew.-% Hydroxid und/oder Oxid zugegeben. Gemäß einer besonderen Abwandlung der vorliegenden Erfindung kann der Anteil an Hydroxid und/oder Oxid so gewählt werden, dass zur Einstellung des pH-Wert der nachfolgenden Hydrolysereaktion gemäß Schritt B) keine weitere Base zugegeben werden muss.

Theoretisch können auch lösliche Amine zur Einstellung des pH-Wertes eingesetzt werden. Allerdings hat sich gezeigt, dass die Verwendung von diesen Aminen einen nachteiligen Einfluss auf die Lebensdauer des zur Hydrolyse gemäß Schritt B) eingesetzten Katalysators haben kann. Neben organischen Verbindungen mit einem Stickstoffatom gehört vorliegend auch Ammoniak (NH₃) zu den Aminen. Daher beträgt der Anteil an löslichen Aminen in der Reaktionsmischung, vorzugsweise höchstens 0,1 Gew.-%, besonders bevorzugt höchstens 0,01 Gew.-% und ganz besonders bevorzugt höchstens 0,001 Gew.-%. Gemäß einem besonderen Aspekt wird kein wesentlicher Anteil an diesen Aminen zur Einstellung des pH-Wertes der Reaktionsmischung hinzu gegeben.

Die Temperatur, bei der die Umsetzung der Carbonylverbindung mit Blausäure erfolgt, kann im Allgemeinen im Bereich von -30 bis 70°C, vorzugsweise im Bereich von -20 bis 60°C, insbesondere im Bereich von -10 bis 50°C und besonders bevorzugt im Bereich von -5 bis 40°C liegen.

Die Umsetzung gemäß Schritt A) zur Bildung eines Hydroxycarbonsäurenitrils kann, je nach Reaktionstemperatur, bei Unter- oder Überdruck durchgeführt werden. Vorzugsweise wird diese Reaktion in einem Druckbereich von 0,5 bis 10 bar, besonders bevorzugt 0,8 bis 3 bar durchgeführt.

Die Reaktionsdauer zur Bildung des Hydroxycarbonsäurenitrils gemäß Schritt A) hängt unter anderem von den eingesetzten Carbonylverbindungen, der Aktivität des Katalysator sowie der Reaktionstemperatur ab, wobei dieser Parameter in weiten Bereichen liegen kann. Bevorzugt liegt die Reaktionszeit zur Umsetzung der Carbonylverbindung mit HCN im Bereich von 30 Sekunden bis 15 Stunden, besonders bevorzugt 10 Minuten bis 5 Stunden und ganz besonders bevorzugt 30 Minuten bis 3 Stunden.

Bei kontinuierlichen Verfahren beträgt die Verweilzeit der Umsetzung gemäß Schritt A) vorzugsweise 30 Sekunden bis 15 Stunden, besonders bevorzugt 10 Minuten bis 5 Stunden und ganz besonders bevorzugt 30 Minuten bis 3 Stunden.

Die nach der Umsetzung gemäß Schritt A) hergestellte Reaktionsmischung wird im Gegensatz zu den Verfahren des Standes der Technik nicht durch Destillation aufgereinigt, bevor die Hydrolyse gemäß Schritt B) durchgeführt wird. Unter Destillation wird hierbei eine Auftrennung der Reaktionsmischung über unterschiedliche Siedepunkte der Mischungsbestandteile verstanden. Hierdurch können erhebliche Effizienzverbesserungen des Verfahrens erzielt werden. Gemäß einer bevorzugten Ausgestaltung der Umsetzung gemäß Schritt A) ist keine Aufreinigung notwendig. Vielmehr kann die nach der Umsetzung gemäß Schritt A) erhaltene Mischung unmittelbar einer Hydrolysereaktion gemäß Schritt B) zugeführt werden.

Die Hydrolyse des in Schritt A) erhaltenen Carbonsäurenitrils wird erfindungsgemäß in Gegenwart eines Mangandioxid umfassenden Katalysators durchgeführt. Vorzugsweise kann die stöchiometrische Zusammensetzung von natürlichem sowie synthetischem Mangandioxid durch den Einbau von Mangan anderer Wertigkeitsstufen in das Kristallgitter im Bereich zwischen MnO_{1,7} bis MnO_{2,0} liegen. Mangandioxid existiert in mehreren allotropen Modifikationen. Sie unterscheiden sich stark in dem Verhalten als Katalysator. Bei Pyrolysit (beta- Mangandioxid), der stabilsten Modifikation, ist die Kristallinität am höchsten ausgeprägt. Die Kristallität ist in den weiteren Modifikationen weniger stark ausgeprägt und geht bis zu amorphen Produkten, zu denen unter anderem alpha- oder delta-MnO₂ gehören. Durch Röntgenbeugung können die Modifikationen zugeordnet werden. Die chemisch und katalytisch besonders aktiven Formen des Mangandioxids können teilweise hydratisiert sein und zusätzlich Hydroxylgruppen enthalten.

Der Mangandioxid umfassende Katalysator kann weitere Verbindungen oder Ionen umfassen. Hierzu gehören insbesondere Alkalimetall- und/oder Erdalkalimetall-Ionen, die bei der Herstellung in das Kristallgitter eingebracht oder auf der Oberfläche des Katalysators abgelagert werden. Zu den bevorzugten Alkalimetall-Ionen gehören insbesondere Lithium-, Natrium- und/oder Kalium-Ionen. Zu den bevorzugten Erdalkalimetall-Ionen gehören insbesondere Calcium- und oder Magnesium-Ionen. Das Gehalt an Alkalimetall- und/oder Erdalkalimetall kann vorzugsweise weniger als 0,6 Atome pro Atom Mangan betragen. Vorzugsweise liegt das Atomverhältnis von Alkalimetall- und/oder Erdalkalimetall zu Mangan im Bereich von 0,01:1 bis 0,5:1, besonders bevorzugt im Bereich von 0,05:1 bis 0,4:1.

Darüber hinaus kann der Mangandioxid umfassende Katalysator Promotoren enthalten, die ebenfalls in das Kristallgitter eingebracht oder auf der Oberfläche des Katalysators abgelagert werden können. Zu den bevorzugten Promotoren gehören unter anderem Ti, Zr, V, Nb, Ta, Cr, Mo, W, Zn, Ga, In, Ge, Sn und Pt. Das Gehalt an Promotoren kann vorzugsweise weniger als 0,3 Atome pro Atom Mangan betragen. Vorzugsweise liegt das Atomverhältnis von Promotor zu Mangan im Bereich von 0,001:1 bis 0,2:1, besonders bevorzugt im Bereich von 0,005:1 bis 0,1:1. Vorzugsweise kann der Mangandioxid umfassende Katalysator 0,01 bis 10 Gew.-%, besonders bevorzugt 0,1 bis 5 Gew.-% Promotoren umfassen, wobei sich diese Größe auf das Gewicht gemessen als Metall oder Metallion bezieht.

Des Weiteren können geeignete Katalysatoren Anteile an SiO₂ oder anderen Bindemitteln umfassen, um die mechanische Stabilität zu erhöhen, wie dies beispielsweise in EP-A-0 956 898 dargelegt ist.

Besonders bevorzugte Katalysatoren umfassen beispielsweise
0,0 bis 25 Gew.-%, insbesondere 0,1 bis 2 Gew.-% SiO₂;
0,1 bis 10 Gew.-%, insbesondere 2 bis 7 Gew.-% K₂O;
0,0 bis 5 Gew.-%, insbesondere 0,2 bis 4 Gew.-% ZrO₂ und
75 bis 99 Gew.-%, insbesondere 85 bis 98 Gew.-% MnO₂. Der Katalysator kann weitere Elemente umfassen, wie dies zuvor dargelegt wurde. Die Zusammensetzung der Katalysatoren kann durch semiquantitative Röntgenfluoreszenzanalyse bestimmt werden.

Bevorzugte Mangandioxid umfassende Katalysatoren weisen im Röntgenspektrum (XRD) gemessen als Pulver mindestens eine Reflexion im Bereich von 32,0 bis 42,0° auf. Die Röntgenspektren können beispielsweise mit einem Xpert pro Gerät der Fa. Panalytical erhalten werden. Diese Reflexion im Bereich von 32,0 bis 42,0° weist besonders bevorzugt die höchste Intensität in Bezug auf die weiteren Intensitäten im Bereich von 20° bis 65° auf, gemessen als Maximum der Reflexion. Besonders bevorzugte Katalysatoren zeigen eine geringe Kristallinität, wobei diese unter anderem aus dem Röntgenspektrum ersehen werden kann. Die Struktur besonders bevorzugter Katalysatoren kann der Strukturnummer 44-0141 oder 72-1982 zugeordnet werden, die in ICDD (International Centre for Diffraction Data) dargelegt ist, wobei die Katalysatoren mit einer Struktur gemäß 44-0141 besonders bevorzugt sind.

Die Alkalimetall- und/oder Erdalkalimetall-Ionen sowie die Promotoren können bei der Herstellung der Katalysatoren beispielsweise in Form von Salzen hinzu gegeben werden. So können insbesondere Halogenide, Nitrate, Sulfate, Carbonate, Phosphate und Hydroxide der zuvor genannten Stoffe eingesetzt werden, wobei vorzugsweise Verbindungen eingesetzt werden, die in Wasser löslich sind.

Vorzugsweise kann der Mangandioxid umfassende Katalysator mindestens 50 Gew.-%, besonders bevorzugt mindestens 80 Gew.-% Mangandioxid mit einer Summenformel MnOₓ umfassen, wobei x im Bereich von 1,7 bis 2,0 liegt.

Gemäß einem besonderen Aspekt der vorliegenden Erfindung kann der Mangandioxid umfassende Katalysator eine spezifische Oberfläche (BET) im Bereich von 50 bis 1000 m² pro g, besonders bevorzugt 100 bis 300 m² pro g und ganz besonders bevorzugt 150 bis 250 m² pro g aufweisen, welche nach der Prüfvorschrift DIN 66131 ermittelt wird.

Je nach Reaktortyp kann der Katalysator beispielsweise in Form von Pulver oder Granulat eingesetzt werden, wobei die Korngröße vielfach vom eingesetzten Reaktionsgefäß abhängig sein kann.

Die Herstellung der zuvor beschriebenen Mangandioxid umfassenden Katalysatoren ist an sich bekannt und beispielsweise in EP-A-0 379 111, EP-A-0 956 898, EP-A-0545697 und EP-A-0 433 611 dargelegt. Bevorzugt können die erfindungsgemäß zu verwendenden Mangandioxid umfassenden Katalysatoren durch Oxidation von Mn²⁺-Salzen, beispielsweise MnSO₄ mit Permanganaten, beispielsweise Kaliumpermanganat erhalten werden (vgl. Biochem.J., 50 S. 43 (1951) und J.Chem.Soc., S. 2189, 1953). Darüber hinaus kann geeignetes Mangandioxid durch elektrolytische Oxidation von Mangansulfat in wässriger Lösung erhalten werden.

Katalysatoren mit Strukturen gemäß 44-0141 können beispielsweise dadurch erhalten werden, dass man eine wässrige Lösung mit 0,71 mol Mn(II)SO₄ (insgesamt 15 Gew.-% Mn²⁺ in Lösung), 0,043 mol Zr(IV)(SO₄)₂, 0,488mol konz. Schwefelsäure und 13,24 mol Wasser bei 70°C zügig zu einer Lösung von 1,09 mol KMnO₄ in 64,5 mol Wasser gibt. Die überstehende Lösung mit dem sich gebildeten Niederschlag kann 3 Stunden auf 90°C temperiert werden. Der Niederschlag kann nachfolgend abfiltriert, viermal mit einem Liter Wasser gewaschen und 12 Stunden bei 110°C getrocknet werden.

Vorzugsweise weist die zum Mangandioxid umfassenden Katalysator gegebene Reaktionsmischung einen pH-Wert im Bereich von 6,0 bis 11,0, bevorzugt 6,5 bis 10,0 und ganz besonders bevorzugt 8,5 bis 9,5 auf. Der pH-Wert ist in diesem Zusammenhang definiert als der negative dekadische Logarithmus der Aktivität der Oxoniumionen (H₃O⁺). Diese Größe ist somit unter anderem von der Temperatur abhängig, wobei sich diese Größe auf die Reaktionstemperatur bezieht. Für die Zwecke der Erfindung ist es vielfach ausreichend diese Größe mit elektrischen Messgeräten (pH-Metern) zu bestimmen, wobei eine Bestimmung bei Raumtemperatur anstatt der Reaktionstemperatur für viele Zwecke ausreichend ist. Die Einstellung des pH-Wertes kann vorzugsweise bereits bei der Herstellung des Hydroxycarbonsäurenitrils gemäß Schritt A) erfolgen, wobei vorzugsweise die zuvor dargelegten Oxide und Hydroxide eingesetzt werden können.

Hierbei ist festzuhalten, dass der Mangandioxid umfassende Katalysator vielfach amphotere Eigenschaften aufweist, daher wird der pH-Wert der Reaktionsmischung bei der Umsetzung durch die Art und Menge des Katalysators stark beeinflusst. Der Ausdruck "die zum Mangandioxid umfassenden Katalysator gegebene Reaktionsmischung" verdeutlicht, dass der pH-Wert ohne Gegenwart des Katalysators gemessen wird. Die weiteren Bestandteile der Reaktionsmischung umfassen beispielsweise Lösungsmittel, Wasser, Carbonsäurenitril usw.

Überraschend wurde festgestellt, dass eine Hydrolyse in Gegenwart von Lithiumionen zu einer besonders langen Lebensdauer des Mangandioxid umfassenden Katalysators führt. Dementsprechend können zur weiteren Verbesserung des erfindungsgemäßen Verfahrens Lithiumverbindungen, insbesondere wasserlösliche Lithiumsalze zur Reaktionsmischung hinzu gegeben werden, beispielsweise LiCl, LiBr, Li₂SO₄, LiOH und/oder Li₂O. Bevorzugt liegt die Konzentration an Lithiumverbindungen im Bereich von 0,001 bis 5 Gew.-%, besonders bevorzugt 0,01 Gew.-% bis 1 Gew.-%. Die Zugabe kann während oder vor der Hydrolysereaktion erfolgen.

Vorzugsweise findet die Hydrolyse des Carbonsäurenitrils zum Carbonsäureamid in Gegenwart eines Oxidationsmittels statt. Geeignete Oxidationsmittel sind in der Fachwelt weithin bekannt. Zu diesen Oxidationsmitteln gehören unter anderem sauerstoffhaltige Gase; Peroxide, beispielsweise Wasserstoffperoxid (H₂O₂), Natriumperoxid, Kaliumperoxid, Magnesiumperoxid, Calciumperoxid, Bariumperoxid, Benzoylperoxid und Diacetylperoxid; Persäuren oder Salze von Persäuren, beispielsweise Perameisensäure, Peressigsäure, Natriumpersulfat, Ammoniumpersulfat und Kaliumpersulfat; sowie Oxosäuren oder Salze von Oxosäuren, beispielsweise Periodsäure, Kaliumperiodat, Natriumperiodat, Perchlorsäure, Kaliumperchlorat, Natriumperchlorat, Kaliumchlorat, Natriumchlorat, Kaliumbromat, Natriumiodat, lodsäure, Natriumhypochlorit, Permanganatsalze, beispielsweise Kaliumpermanganat, Natriumpermanganat und Lithiumpermanganat, und Salze der Chromsäure, beispielsweise Kaliumchromat, Natriumchromat und Ammoniumchromat.

Die Menge des eingesetzten Oxidationsmittels kann in einem weiten Bereich liegen, wobei die Reaktanden und Produkte nicht durch das Oxidationsmittel oxidiert werden sollten. Daher kann die Oxidationsempfindlichkeit dieser Stoffe den Einsatz der Oxidationsmittel begrenzen. Die untere Grenze ergibt sich aus der zu erzielenden Verbesserung der Haltbarkeit des Katalysators. Vorzugsweise liegt das molare Verhältnis von Oxidationsmittel zu Carbonsäurenitril im Bereich von 0,001:1 bis 2:1, besonders bevorzugt 0,01:1 bis 1,5:1.

Diese Oxidationsmittel können beispielsweise als Lösung und/oder als Gas der Reaktionsmischung zugegeben werden. Besonders bevorzugt werden als Oxidationsmittel Gase eingesetzt, die Sauerstoff umfassen. Hierbei kann das Gas molekularen Sauerstoff (O₂) oder Ozon (O₃) enthalten. Des Weiteren kann das als Oxidationsmittel eingesetzte Gas weitere Gase, insbesondere Inertgase, wie Stickstoff oder Edelgase enthalten. Gemäß einem besonderen Aspekt kann das Gas vorzugsweise 50 bis 98 Vol.-% Inertgas und 2 bis 50 Vol.-% molekularen Sauerstoff (O₂) umfassen. Zu den bevorzugten Gasen gehört insbesondere Luft. Des Weiteren kann auch ein Gas eingesetzt werden, das weniger als 20 Vol.-%, insbesondere weniger als 10 Vol.-% molekularen Sauerstoff enthält, wobei diese Gase im Allgemeinen mindestens 1 Vol.-% vorzugsweise mindestens 2 Vol.-% Sauerstoff enthalten.

Vorzugsweise kann die durch die Reaktionsmischung hindurchgeleitete Menge an Sauerstoff umfassendem Gas im Bereich von 1 bis 5000, besonders bevorzugt im Bereich von 10 bis 1000 Liter/Stunde, bezogen auf 1 kg Mangandioxid umfassenden Katalysator liegen.

Das Wasser, welches zur Hydrolyse des Carbonsäurenitrils notwendig ist, kann vielfach als Lösungsmittel eingesetzt werden. Vorzugsweise beträgt das molare Verhältnis von Wasser zu Carbonsäurenitril mindestens 1, besonders bevorzugt liegt das molare Verhältnis von Wasser zu Carbonsäurenitril im Bereich von 0,5:1-25:1 und ganz besonders bevorzugt im Bereich von 1:1-10:1.

Das zur Hydrolyse eingesetzte Wasser kann einen hohen Reinheitsgrad aufweisen. Allerdings ist diese Eigenschaft nicht zwingend. So kann neben Frischwasser auch Brauchwasser oder Prozesswasser eingesetzt werden, welches mehr oder minder hohe Mengen an Verunreinigungen umfasst. Dementsprechend kann auch recyceltes Wasser zur Hydrolyse verwendet werden.

Des Weiteren können weitere Bestandteile in der Reaktionsmischung zur Hydrolyse des Carbonsäurenitrils vorhanden sein. Hierzu gehören u.a. Carbonylverbindungen, wie Aldehyde und Ketone, insbesondere jene, die zur Herstellung von bevorzugt als Carbonsäurenitril einzusetzenden Cyanhydrinen verwendet wurden. Beispielsweise kann Aceton und / oder Acetaldehyd in der Reaktionsmischung enthalten sein. Dies wird beispielsweise in US 4018829-A beschrieben. Die Reinheit der zugegebenen Aldehyde und/oder Ketone ist im Allgemeinen nicht besonders kritisch. Dementsprechend können diese Stoffe Verunreinigungen, insbesondere Alkohole, beispielsweise Methanol, Wasser und/oder α-Hydroxyisobuttersäuremethylester (HIBSM) enthalten. Die Menge an Carbonylverbindungen, insbesondere Aceton und / oder Acetaldehyd kann in der Reaktionsmischung in weiten Bereichen eingesetzt werden. Vorzugsweise wird die Carbonylverbindung in einer Menge im Bereich von 0,1-6 Mol, vorzugsweise 0,1-2 Mol pro Mol Carbonsäurenitril eingesetzt. Gemäß einer besonderen Abwandlung der vorliegenden Erfindung kann diese Carbonylverbindung vollständig bei Schritt A) zugegeben werden, so dass dieser Überschuss im Kreis geführt wird.

Die Temperatur, bei der die Hydrolysereaktion erfolgt, kann im Allgemeinen im Bereich von 10-150°C, vorzugsweise im Bereich von 20-100°C und besonders bevorzugt im Bereich von 30-80°C liegen.

Die Hydrolysereaktion kann, je nach Reaktionstemperatur, bei Unter- oder Überdruck durchgeführt werden. Vorzugsweise wird diese Reaktion in einem Druckbereich von 0,1-10 bar, besonders bevorzugt 0,5 bis 5 bar durchgeführt.

Die Reaktionsdauer der Hydrolysereaktion hängt unter anderem von den eingesetzten Carbonsäurenitrilen, der Aktivität des Katalysator sowie der Reaktionstemperatur ab, wobei dieser Parameter in weiten Bereichen liegen kann. Bevorzugt liegt die Reaktionszeit der Hydrolysereaktion im Bereich von 30 Sekunden bis 15 Stunden, besonders bevorzugt 15 Minuten bis 10 Stunden und ganz besonders bevorzugt 60 Minuten bis 5 Stunden.

Bei kontinuierlichen Verfahren beträgt die Verweilzeit vorzugsweise 30 Sekunden bis 15 Stunden, besonders bevorzugt 15 Minuten bis 10 Stunden und ganz besonders bevorzugt 60 Minuten bis 5 Stunden.

Die Beladung des Katalysators mit Carbonsäurenitril kann in einem weiten Bereich liegen. Vorzugsweise werden 0,01 bis 2,0, besonders bevorzugt 0,05 bis 1,0 und ganz besonders bevorzugt 0,1 bis 0,4 g Carbonsäurenitril pro g Katalysator pro Stunde eingesetzt.

Die Reaktion gemäß Schritt B) kann beispielsweise in einem Festbettreaktor oder in einem Suspensionsreaktor durchgeführt werden. Falls Gase als Oxidationsmittel eingesetzt werden, können insbesondere so genannte Rieselbettreaktoren eingesetzt werden, die einen guten Kontakt von Gas, Feststoff und Flüssigkeit ermöglichen. Bei Rieselbettreaktoren ist der Katalysator in Form eines Festbettes angeordnet. Hierbei kann der Rieselbettreaktor (Trickle bed reactor) in Gleichstrom- oder Gegenstromfahrweise betrieben werden.

Die nach Schritt B) erhaltene Reaktionsmischung weist im Allgemeinen neben dem gewünschten Carbonsäureamid weitere Bestandteile auf, insbesondere nicht umgesetztes Carbonsäurenitril oder Blausäure sowie überschüssig eingesetzte Carbonylverbindung, insbesondere Aceton und / oder Acetaldehyd, sowie überschüssig eingesetztes Wasser.

Dementsprechend wird die Reaktionsmischung aufgetrennt, um die bereits eingesetzten Edukte erneut umsetzen zu können. Die nach Schritt B) erhaltene Reaktionsmischung wird durch eine zweistufige Destillation aufgereinigt. Die nach Schritt B) erhaltene Reaktionsmischung umfasst im Allgemeinen noch Anteile an Hydroxycarbonsäurenitril. Der Siedepunkt des Hydroxycarbonsäurenitrils liegt höher als der von Wasser. Probleme, die hiermit verbunden sind, können leicht durch eine Zersetzung des Hydroxycarbonsäurenitrils in eine Carbonylverbindung und Blausäure gelöst werden. Diese Zersetzung kann beispielsweise durch Gegenwart einer Base, vorzugsweise eines Anionenaustauschers katalysiert werden, die bzw. der beispielsweise im Sumpf der Destille vorgesehen ist.

Die Temperatur, bei der Wasser von Carbonylverbindung und Blausäure gemäß Schritt b) getrennt wird, kann im Allgemeinen im Bereich von 50 bis 150°C, vorzugsweise im Bereich von 70 bis 120°C und besonders bevorzugt im Bereich von 90 bis 110°C liegen. Der zweite Destillationsschritt b) kann vorzugsweise in einem Druckbereich von 0,2 bis 5 bar, besonders bevorzugt 0,7 bis 1,5 bar durchgeführt werden. Aus der nach Schritt B) erhaltenen Reaktionsmischung wird in einem ersten Destillationsschritt a') zunächst die Carbonylverbindung und Blausäure über Kopf abgetrennt. Hydroxycarbonsäurenitril wird hierbei zunächst in Carbonylverbindung und Blausäure aufgetrennt und auf diese Weise ebenfalls über Kopf aus der Mischung entfernt. Die auf diese Weise gewonnenen Blausäure und Carbonylverbindung kann zur Herstellung von Hydroxycarbonsäurenitril gemäß Schritt A) eingesetzt werden. Die aus dem Sumpf erhaltene Mischung umfasst Wasser und Carbonsäureamid. Diese Mischung wird in einem zweiten Schritt b') aufgetrennt, wobei das Carbonsäureamid von Wasser mit Vorteil in einer mehrstufigen Eindampfung, auch mehrstufige Verdampfung genannt, getrennt werden kann. Hierbei wird die durch Primärenergie erzeugte Brüdenmenge in einer zweiten Stufe auf niedrigerem Druckniveau als Heizmedium für die Flüssigphase eingesetzt. Dieses Prinzip lässt sich über mehrere Stufen zur Energieeinsparung fortsetzen. Mit Vorteil umfasst eine mehrstufige Eindampfung zwei bis vier dieser Trennstufen. Das Prinzip der mehrstufigen Eindampfung ist unter anderem in H.G. Hirschberg, Handbuch der Verfahrenstechnik und des Anlagenbau, Springer 1999 detaillierter dargelegt, wobei zu Offenbarungszwecken auf dieses Dokument verwiesen wird. Diese Ausgestaltung ermöglicht überraschend eine besonders energieeffiziente Aufreinigung der in Schritt B) erhaltenen Reaktionsmischung. Das erhaltene Wasser kann zur Hydrolyse gemäß Schritt B) verwendet werden.

Der erste Destillationsschritt a') zur Auftrennung der gemäß Schritt B) erhaltenen Reaktionsmischung in Carbonsäureamid und Wasser und eine Mischung, umfassend Carbonylverbindung und Blausäure, kann vorzugsweise bei einer Temperatur im Bereich von 50 bis 170°C, besonders bevorzugt im Bereich von 90 bis 120°C durchgeführt werden. Der Druck liegt bei diesem Schritt a') bevorzugt im Bereich von 0,4 bis 5 bar, besonders bevorzugt im Bereich von 0,7 bis 2 bar. Die hierin dargelegten Destillationstemperaturen beziehen sich insbesondere auf die Sumpftemperatur.

Die Temperatur, bei der Wasser und Carbonsäureamid gemäß Schritt b') getrennt werden, kann im Allgemeinen im Bereich von 90 bis 260°C, vorzugsweise im Bereich von 100 bis 180°C liegen. Der zweite Destillationsschritt b') kann vorzugsweise in einem Druckbereich von 10 mbar bis 20 bar, besonders bevorzugt 100 mbar bis 10 bar durchgeführt werden. Hohe Druckwerte gelten insbesondere für die ersten Stufen einer mehrstufigen Eindampfung.

Überraschende Vorteile hinsichtlich der Standzeit der Anlage und der Lebensdauer des Katalysators können insbesondere dadurch erzielt werden, dass über den Rücklauf in die zur Trennung von Wasser und Blausäure eingesetzte Destille eine Zusammensetzung eingeleitet wird, die die in Schritt A) eingesetzte Carbonylverbindung umfasst, wobei die in den Rücklauf eingeleitete Zusammensetzung einen geringeren Anteil an HCN aufweist, als die über Kopf der Destille entnommene Zusammensetzung. Der Gewichtsanteil an HCN in der Zusammensetzung, die in den Rücklauf eingeleitet wird, beträgt vorzugsweise höchstens 60 %, besonders bevorzugt höchstens 40 % und ganz besonders bevorzugt höchstens 10 %, der auf das Gewicht bezogenen Menge an HCN, die in der über Kopf der Destille entnommenen Zusammensetzung enthalten ist. Besonders bevorzugt enthält die in den Rückfluss der Destille eingeleitete Zusammensetzung im Wesentlichen kein HCN. In diesem Zusammenhang ist festzuhalten, dass sich aus der abgetrennten Carbonylverbindung und Blausäure Hydroxycarbonsäurenitril bilden kann, wobei diese ebenfalls nicht in die Destille zurückgeführt werden sollte. Dementsprechend beziehen sich die zuvor dargelegten Angaben bezüglich der Menge an HCN, die in die Destille zurückgeleitet wird, auf die Summe der Menge an freiem HCN und an HCN, welches in Form von Hydroxycarbonsäurenitril gebunden ist. Die Menge an gebundenem HCN kann durch Zersetzung des Hydroxycarbonsäurenitrils bestimmt werden. Dementsprechend enthält die in den Rückfluss der Destille eingeleitete Zusammensetzung im Wesentlichen auch kein Hydroxycarbonsäurenitril.

Gemäß einer bevorzugten Abwandlung der vorliegenden Erfindung wird die bei der Auftrennung von Wasser und Blausäure abgetrennte Carbonylverbindung zur Herstellung eines Hydroxycarbonsäurenitrils gemäß Schritt A) eingesetzt. Dementsprechend wird das Kopfprodukt des entsprechenden Destillationsschritts bevorzugt zur Herstellung des in Schritt B) eingesetzten Hydroxycarbonsäurenitrils verwendet. Entsprechend kann das erhaltene Wasser zur Hydrolyse des Hydroxycarbonsäurenitrils gemäß Schritt B) eingesetzt werden.

Eine weitere Verbesserung der Lebensdauer des Katalysators kann insbesondere durch die Verwendung einer Kolonne mit einer hohen Trennleistung, insbesondere bei der Auftrennung von Wasser und Blausäure erzielt werden. Dementsprechend wird hierfür vorzugsweise eine Destillationskolonne verwendet, die zwei oder mehr Trennstufen besitzt. Als Anzahl der Trennstufen wird in der vorliegenden Erfindung die Anzahl der Böden bei einer Bodenkolonne oder die Anzahl der theoretischen Trennstufen im Fall einer Packungskolonne oder eine Kolonne mit Füllkörpern bezeichnet.

Beispiele für eine mehrstufige Destillationskolonne mit Böden beinhalten solche wie Glockenböden, Siebböden, Tunnelböden, Ventilböden, Schlitzböden, Sieb-Schlitzböden, Sieb-Glockenböden, Düsenböden, Zentrifugalböden, für eine mehrstufige Destillationskolonne mit Füllkörpern solche wie Raschig-Ringe, Lessing-Ringe, Pall-Ringe, Berl-Sättel, Intalox Sättel und für eine mehrstufige Destillationskolonne mit Packungen wie solche vom Typ Mellapak (Sulzer), Rombopak (Kühni), Montz-Pak (Montz) und Packungen mit Katalysatortaschen, beispielsweise Kata-Pak.

Eine Destillationskolonne mit Kombinationen aus Bereichen von Böden, aus Bereichen von Füllkörpern oder aus Bereichen von Packungen kann ebenso verwendet werden.

Durch die Verwendung einer Destillationskolonne mit einer hohen Trennleistung sowie eines Rücklaufs mit geringem Blausäuregehalt kann der Anteil an HCN in der wässrigen Phase, die zur Hydrolyse eingesetzt wird, sehr gering gehalten werden. Vorzugsweise wird zur Hydrolyse eine wässrige Phase zurückgeführt, die nur äußerst geringe Anteile an Blausäure umfasst, wobei insbesondere Werte kleiner 1 Gew.-%, besonders bevorzugt kleiner 0,5 Gew.-% und besonders bevorzugt kleiner 0,1 Gew.-% Blausäure, bezogen auf die zurückgeführte wässrige Phase erzielt werden können.

Überraschende Vorteile können insbesondere dadurch erzielt werden, dass Menge an Carbonylverbindung, die im Destillationsschritt zur Auftrennung von Wasser und Blausäure zugegeben wird, so gewählt wird, dass diese zur Herstellung der in Schritt A) vorgesehenen Menge an Hydroxycarbonsäurenitril ausreicht. Dementsprechend wird bevorzugt die zur Umsetzung von HCN benötigte Carbonylverbindung vollständig in den Rücklauf der Destille gegeben, die zur Destillation einer Mischung eingesetzt wird, die Wasser, HCN und die in Schritt A) eingesetzte Carbonylverbindung umfasst. Je nach Ausgestaltung kann dies bei der ersten oder zweiten Destillation erfolgen.

Des Weiteren kann die gereinigte Carbonsäureamid umfassende Reaktionsmischung durch lonenaustauschsäulen von weiteren Bestandteilen gereinigt werden.

Hierzu können insbesondere Kationenaustauscher und Anionenaustauscher eingesetzt werden. Hierfür geeignete lonenaustauscher sind an sich bekannt. Beispielsweise können geeignete Kationenaustauscher durch Sulfonierung von Styrol-Divinylbenzolcopolymeren erhalten werden. Basische Anionenaustauscher umfassen quaternäre Ammoniumgruppen, die kovalent an Styrol-Divinylbenzolcopolymere gebunden sind.

Die Aufreinigung von α-Hydroxycarbonsäureamiden ist u.a. in EP-A-0686623 detaillierter beschrieben.

Die Hydrolysereaktion der vorliegenden Erfindung kann insbesondere als Zwischenschritt in Verfahren zur Herstellung von Methacrylsäure (2-Methylpropensäure) sowie deren Ester dienen. Dementsprechend stellt die vorliegende Erfindung auch ein Verfahren zur Herstellung von Methylmethacrylat zur Verfügung, welches einen Hydrolyseschritt gemäß einem Verfahren der vorliegenden Erfindung aufweist. Verfahren, die einen Hydrolyseschritt von Cyanhydrinen zur Herstellung von (Meth)acrylsäure und/oder Alkyl(meth)acrylaten aufweisen können, sind unter anderem in EP-A-0 406 676, EP-A-0 407 811, EP-A-0 686 623 und EP-A-0 941 984 dargelegt. Alkyl(meth)acrylate können aus Carbonylverbindungen, Blausäure und Alkoholen auf einfache und kostengünstige Weise durch Verfahren erhalten werden, die die folgenden Schritte umfassen:
A) Bildung mindestens eines Cyanhydrins durch Umsetzung mindestens einer Carbonylverbindung mit Blausäure;
B) Hydrolyse des Cyanhydrins bzw. der Cyanhydrine unter Bildung mindestens eines α-Hydroxycarbonsäureamids;
C) Alkoholyse des α-Hydroxycarbonsäureamids bzw. der α-Hydroxycarbonsäureamide, wobei mindestens ein α-Hydroxycarbonsäurealkylester erhalten wird;
D) Umesterung des α-Hydroxycarbonsäurealkylesters bzw. der α-Hydroxycarbonsäurealkylester mit (Meth)acrylsäure, wobei mindestens ein Alkyl(meth)acrylat und mindestens eine α-Hydroxycarbonsäure gebildet werden;
E) Dehydratisierung der α-Hydroxycarbonsäure bzw. der α-Hydroxycarbonsäuren, wobei (Meth)acrylsäure gebildet wird.

Die Schritte A) und B) wurden zuvor ausführlich dargelegt. Im nächsten Schritt C) kann das so erhaltene α-Hydroxycarbonsäureamid zum α-Hydroxycarbonsäurealkylester umgesetzt werden. Dies kann beispielsweise durch die Verwendung von Alkylformiaten erfolgen. Insbesondere geeignet ist Methylformiat oder eine Mischung von Methanol und Kohlenmonooxid, wobei diese Reaktion beispielhaft in EP-A-0407811 beschrieben ist.

Bevorzugt erfolgt die Umsetzung des α-Hydroxycarbonsäureamids durch Alkoholyse mit einem Alkohol, der vorzugsweise 1-10 Kohlenstoffatome, besonders bevorzugt 1 bis 5 Kohlenstoffatome umfasst. Bevorzugte Alkohole sind u.a. Methanol, Ethanol, Propanol, Butanol, insbesondere n-Butanol und 2-Methyl-1-propanol, Pentanol, Hexanol, Heptanol, 2-Ethylhexanol, Octanol, Nonanol und Decanol. Besonders bevorzugt wird als Alkohol Methanol und / oder Ethanol eingesetzt, wobei Methanol ganz besonders bevorzugt ist. Die Umsetzung von Carbonsäureamiden mit Alkoholen, um Carbonsäureester zu erhalten, ist allgemein bekannt.

Das Molverhältnis von α-Hydroxycarbonsäureamid zu Alkohol, beispielsweise α-Hydroxyisobuttersäureamid zu Methanol, ist an sich nicht kritisch, wobei dieses vorzugsweise im Bereich von 3:1 bis1:20 liegt. Ganz besonders zweckmäßig liegt dieses Verhältnis im Bereich von 2:1 bis 1:15 und besonders bevorzugt im Bereich von 1:1 bis 1:10.

Die Reaktionstemperatur kann ebenfalls in weiten Bereichen liegen, wobei die Reaktionsgeschwindigkeit mit zunehmender Temperatur im Allgemeinen zunimmt. Die obere Temperaturgrenze ergibt sich im Allgemeinen aus dem Siedepunkt des eingesetzten Alkohols. Vorzugsweise liegt die Reaktionstemperatur im Bereich von 40-300°C, besonders bevorzugt 160-240°C. Die Reaktion kann, je nach Reaktionstemperatur, bei Unter- oder Überdruck durchgeführt werden. Vorzugsweise wird diese Reaktion in einem Druckbereich von 0,5-200 bar, besonders zweckmäßig in einem Bereich von 1 bis 100 bar und besonders bevorzugt 5 bis 30 bar durchgeführt. Die Umsetzung zwischen alpha-Hydroxycarbonsäureamid und Alkohol kann in einem Druckreaktor durchgeführt werden.

Hierunter ist grundsätzlich ein Reaktionsraum zu verstehen, der es gestattet während der Umsetzung einen Überdruck aufrechtzuerhalten. Überdruck meint in diesem Zusammenhang ein Druck größer als Atmosphärendruck, d.h. insbesondere größer als 1 bar. Vorzugsweise kann der Druck in einem Bereich von größer 1 bar bis kleiner gleich 100 bar liegen. Dementsprechend kann der Druck sowohl während der Umsetzung /Alkoholyse des alpha-Hydroxycarbonsäureamids als auch während der Abtrennung/Entfernung des Ammoniaks aus dem Produktgemisch größer als Atmosphärendruck oder größer als 1 bar sein. Daher kann der bei der Umsetzung entstehende Ammoniak aus der Mischung unter einem Druck von größer 1 bar abdestilliert werden, wobei auf den Einsatz von Hilfsmitteln wie Strip-Gas zur destillativen Entfernung des Ammoniak vollständig verzichtet werden kann.

Die Produktmischung kann nicht nur an Ammoniak sondern auch an nicht umgesetztem Alkohol abgereichert werden. Gerade für den Fall, dass man zur Alkoholyse Methanol verwendet resultiert ein Produktgemisch unter anderem mit den prinzipiell sehr schwer voneinander trennbaren Komponenten Ammoniak und Methanol. Im einfachsten Fall entfernt man zur Abreicherung des Produktgemisches an Ammoniak und Alkohol die besagten beiden Komponenten direkt als Stoffgemisch aus dem Produktgemisch. Die beiden Stoffe werden dann einer Trennoperation zum Beispiel einer Rektifikation unterworfen. Des Weiteren können die beiden Komponenten Alkohol (Methanol) und Ammoniak in einem Vorgang vom Produktgemisch getrennt und dabei zugleich die beiden Bestandteile Ammoniak und Alkohol (Methanol) noch voneinander separiert werden.

Der Umsetzungsschritt und die Entfernung des Ammoniaks/Alkohols aus der Produktmischung können räumlich voneinander getrennt und in unterschiedlichen Aggregaten durchgeführt werden. Zu diesem Zweck kann man beispielsweise ein oder mehrere Druckreaktoren vorsehen und diese mit einer Druckdestillationskolonne verbinden. Hierbei handelt es sich um einen oder mehrere Reaktoren, die außerhalb der Kolonne in einem separaten Bereich angeordnet sind.

Bevorzugt kann ein kontinuierliches Verfahren zur Herstellung von alpha-Hydroxycarbonsäureestern angewendet werden, bei welchen man als Edukte alpha-Hydroxycarbonsäureamid mit einem Alkohol in Gegenwart eines Katalysators unter Erhalt einer Produktmischung umsetzt, die alpha-Hydroxycarbonsäureester, Ammoniak, nicht umgesetztes alpha-Hydroxycarbonsäureamid sowie Alkohol und Katalysator aufweist; wobei man
a') Eduktstöme umfassend als Edukte ein alpha-Hydroxycarbonsäureamid, einen Alkohol und einen Katalysator in einen Druckreaktor einspeist;
b') die Eduktströme im Druckreaktor bei einem Druck im Bereich von größer 1 bar bis 100 bar miteinander umsetzt;
c') die aus Schritt b') resultierende Produktmischung aufweisend alpha-Hydroxycarbonsäureester, nicht umgesetztes alpha-Hydroxycarbonsäureamid und Katalysator aus dem Druckreaktor ausschleust; und
d') die Produktmischung an Alkohol und Ammoniak abreichert, wobei Ammoniak bei einem Druck, der ständig größer als 1 bar gehalten wird, abdestilliert wird.

Hierbei kann eine besonders zweckmäßige Verfahrensabwandlung vorsehen, dass man
b1) die Edukte im Druckreaktor bei einem Druck im Bereich von 5 bar bis 70 bar miteinander umsetzt;
b2) die aus Schritt b1) resultierende Produktmischung auf einen Druck kleiner als den Druck im Druckreaktor und größer als 1 bar entspannt;
c1) die aus Schritt b2) resultierende entspannte Produktmischung in eine Destillationskolonne einspeist;
c2) in der Destillationskolonne Ammoniak und Alkohol über Kopf abdestilliert, wobei der Druck in der Destillationskolonne im Bereich von größer 1 bar bis kleiner gleich 10 bar gehalten wird; und
d1) die aus Schritt c2) resultierende an Ammoniak und Alkohol abgereicherte Produktmischung aufweisend alpha-Hydroxycarbonsäureester, nicht umgesetztes alpha-Hydroxycarbonsäureamid und Katalysator aus der Kolonne ausschleust.

Gemäß dieser bevorzugten Verfahrensvariante finden Umsetzung der Edukte und Abtrennung von Ammoniak/Alkohol in zwei verschiedenen räumlich voneinander getrennten Aggregaten statt. Mit anderen Worten, Reaktor/Reaktionsraum und Trennaggregat für die Abtrennung von Ammoniak/Alkohol vom Produktgemisch sind voneinander separiert. Dies hat den Vorteil, dass man für die Reaktion/Umsetzung der Edukte und die anschließende Abtrennung von Ammoniak/Alkohol unterschiedliche Druckbereiche anwenden kann. Durch die Auftrennung des Verfahrens in einen Umsetzungsschritt im Druckreaktor unter höherem Druck als in einem Trennschritt in einer Druckkolonne, wobei beide Schritte unter Überdruck, d.h. größer als 1 bar geführt werden, kann die Trennwirkung überraschend nochmals signifikant verbessert und die Effizienz der Abtrennung des Ammoniak/Alkohol-Gemisches vergrößert werden.

Die genannten Qualitätsmerkmale lassen sich noch weiter verbessern, indem man die Reaktion im Druckreaktor einmal oder mehrmals mit der im Sumpf der Trennkolonne (Druckdestillationskolonne) an Ammoniak und Alkohol abgereicherten Produktmischung wiederholt, wobei der Umsetzungsschritt auf eine Mehrzahl von Druckreaktoren verlagert wird, die in Reihe geschaltet sind.

Ganz besonders bevorzugt wird insofern eine Verfahrensvariante, die dadurch gekennzeichnet ist, dass man
e) die in Schritt d1) ausgeschleuste Produktmischung auf einen Druck in Bereich von 5 bis 70 bar verdichtet;
f) die dergestalt gemäß Schritt e) verdichtete Mischung zur Umsetzung in einen weiteren Druckreaktor einspeist und erneut reagieren lässt; und
g) die Schritte b2), c1), c2) und d1) gemäß der vorgenannten Aufzählung wiederholt.

Demzufolge ist es von besonderem Interesse, dass man die an Ammoniak und Alkohol abgereicherte Mischung aus einem Boden oberhalb des Sumpfes der ersten Destillationskolonne entnimmt, auf einen Druck größer als in der Destillationskolonne verdichtet und anschließend in einen zweiten Druckreaktor einspeist, von wo nach erneuter Umsetzung unter Einwirkung von erhöhtem Druck und Temperatur und unter Erhalt einer zweifach umgesetzten Produktmischung diese wiederum auf einen Druck kleiner als im zweiten Druckreaktor und größer als 1 bar entspannt wird und danach in die erste Destillationskolonne unterhalb des Bodens von dem die Einspeisung in den zweiten Druckreaktor erfolgte aber oberhalb des Sumpfes der ersten Destillationskolonne zurückgeführt wird, wo unter Erhalt einer zweifach an Ammoniak und Alkohol abgereicherten Mischung Ammoniak und Alkohol erneut über Kopf abdestilliert werden.

Dieser Verfahrensschritt kann beliebig wiederholt werden, besonders günstig sind zum Beispiel drei bis vier Wiederholungen. Insofern bevorzugt ist ein Verfahren, dass sich dadurch kennzeichnet, dass man die Umsetzung im Druckreaktor, die Entspannung der umgesetzten Mischung, die Einspeisung in die erste Destillationskolonne, die Abreicherung an Ammoniak und Alkohol in der ersten Destillationskolonne, die Entnahme der abgereicherten Mischung, Verdichtung und Einspeisung der abgereicherten Mischung in einen weiteren Druckreaktor mehrfach wiederholt, wobei eine je nach Anzahl n der in Serie geschalteten Druckreaktoren eine n-fach an Ammoniak und Alkohol abgereicherte Produktmischung am Boden der Druckdestillationskolonne erhalten wird. Hierbei kann n eine positive ganze Zahl größer null sein. Bevorzugt liegt n im Bereich von 2 bis 10.

Eine zweckmäßige Verfahrensabwandlung sieht vor, dass man die zuvor erwähnten und definierten Schritte e) bis g) mehrfach wiederholt.

Ganz spezielle Verfahrensvarianten umfassen, dass man die Umsetzung und Abreicherung viermal unter Einsatz von vier in Reihe geschalteten Druckreaktoren unter Erhalt einer vierfach an Ammoniak und Alkohol abgereicherten Produktmischung durchführt. Diese Verfahrensvariante kennzeichnet sich demnach dadurch, dass man die Schritte e) bis g) noch wenigstens zweimal wiederholt, so dass die Umsetzung insgesamt in wenigstens vier hintereinander geschalteten Druckreaktoren durchgeführt wird.

Für die angegebene Verfahrensvariante haben sich verschiedene Temperaturbereiche in Kolonne und Reaktor als besonders zweckmäßig erwiesen.

Beispielsweise kann die Druckdestillationskolonne im Allgemeinen und bevorzugt eine Temperatur im Bereich von etwa 50°C bis etwa 160°C aufweisen. Die exakte Temperatur wird typisch durch das siedende System in Abhängigkeit der herrschenden Druckbedingungen eingestellt.

Die Temperatur im Reaktor liegt bevorzugt im Bereich von etwa 120°C - 240°C. Dabei ist es ganz besonders zweckmäßig, die Temperatur von Reaktor zu Reaktor abzusenken, beispielsweise in Schritten im Bereich von 3 - 15°C, bevorzugt 4 - 10°C und ganz besonders zweckmäßig in Schritten von 5°C. Hierdurch wird die Selektivität der Umsetzung positiv beeinflusst.

Eine weitere Maßnahme zur Steigerung der Selektivität kann auch darin bestehen, das Reaktorvolumen von Reaktor zu Reaktor zu verringern. Mit abnehmendem Reaktorvolumen bei zunehmendem Umsatz erhält man ebenfalls eine verbesserte Selektivität.

Wie bereits zuvor erwähnt, ist es günstig, die aus der Druckdestillationskolonne zu entnehmende Produktmischung an bestimmten Stellen der Kolonne zu entnehmen. Hierbei wird zur Orientierung als relative Ortsangabe der Abstand der Entnahmestelle zum Sumpf (Kolonnenboden) der Kolonne verwendet. Besonders zweckmäßig geht man so vor, dass die entspannte Produktmischung gemäß Schritt c1) nach jeder erneuten Umsetzung in einem Druckreaktor näher benachbart zum Sumpf der Destillationskolonne eingespeist wird, bezogen auf die Einspeisestelle der Einspeisung des vorherigen Schrittes c1).

Neben der beschriebenen Variante, bei welcher die Umsetzung des alpha-Hydroxycarbonsäureamids mit dem Alkohol von der Entfernung des dabei unter anderem resultierenden Ammoniaks in zwei räumlich voneinander getrennten aber verbundenen Aggregaten durchgeführt wird, kann es in einer weiteren Verfahrensmodifikation bevorzugt sein, den Umsetzungsschritt und den Entfernungsschritt in einem einzigen Aggregat vorzunehmen. Druckreaktor und Druckdestillationskolonne werden dabei in einem einzigen Aggregat verwirklicht, fallen quasi zusammen.

Der in der zuvor beschriebenen erfindungsgemäßen Variante einzuhaltende Druckbereich, vorzugsweise in einer als Reaktor dienenden Reaktivdestillationskolonne, ist über weite Bereiche variabel. Eine bevorzugte Ausführungsform umfasst dabei, dass man die Schritte a) bis c) simultan in einer Reaktivdestillationskolonne bei einem Druck im Bereich von 5 bar bis 40 bar ausführt. Besonders zweckmäßig ist ein Verfahren, das sich dadurch auszeichnet, dass man die Schritte a) bis c) simultan in einer Reaktivdestillationskolonne bei einem Druck im Bereich von 10 bar bis 30 bar ausführt.

In einer bevorzugten Variante des Verfahrens wird die Umsetzung der Edukte in einer als Druckkolonne ausgelegten Reaktivdestillationskolonne durchgeführt und das entstehende Ammoniak während der Umsetzung kontinuierlich über den Kopf der Kolonne abdestilliert. Hierdurch wird der überraschende Effekt erzielt, dass sich Ammoniak ohne dass man den Druck verringern müsste auf einfachste Weise abtrennen und in hoher Reinheit wiedergewinnen lässt. Weiterhin von besonderem Interesse ist dabei eine Variante, bei der man Ammoniak unter Druck über den Kopf der Kolonne abdestilliert und den Alkohol über den Sumpf oder über einen Seitenstrom aus der Kolonne entfernt. Durch eine entsprechend ausgelegte Trennwirkung der Reaktivdestillationskolonne erreicht man also eine unmittelbare Separation von Ammoniak und Alkohol. Es kann gemäß einer Variante jede mehrstufige druckfeste Reaktivdestillationskolonne verwendet werden, die bevorzugt zwei oder mehr Trennstufen besitzt. Derartige Reaktivdestillen werden im Zusammenhang mit Schritt D) näher erläutert, wobei diese auch für die Umsetzung des Carbonsäureamids mit einem Alkohol eingesetzt werden können.

Die an Ammoniak abgereicherte Produktmischung weist unter anderem den angestrebten alpha-Hydroxycarbonsäureester auf. Zur weiteren Isolierung und Reinigung des Esters kann man in zweckmäßiger Verfahrensabwandlung die an Ammoniak abgereicherten Produktmischung über den Sumpf der Reaktivdestillationskolonne abziehen und einer weiteren zweiten Destillationskolonne zuführen, wo man unter Erhalt einer an sowohl Ammoniak als auch Alkohol abgereicherten Mischung den Alkohol über den Kopf der Kolonne abdestilliert und bevorzugt in einen Reaktor zurückführt.

Zur weiteren Isolierung und Gewinnung des alpha-Hydroxycarbonsäureesters aus der an Ammoniak und Alkohol abgereicherten Mischung ist dann ein Verfahren bevorzugt, bei dem man die an Ammoniak und Alkohol abgereicherte Mischung über den Sumpf der weiteren Destillationskolonne ausschleust und noch einer weiteren Destillationskolonne zuführt, in welcher man den alpha-Hydroxycarbonsäureester über Kopf abdestilliert und die so erhaltene an Ammoniak, Alkohol und alpha-Hydroxycarbonsäureester abgereicherte Mischung, gegebenenfalls nach weiteren Reinigungsschritten, in den Reaktor zurückführt. Das über Kopf der Kolonne gewonnene alpha-Hydroxycarbonsäureesterprodukt ist hochrein und kann beispielsweise äußerst vorteilhaft weiteren Reaktionsschritten zur Gewinnung von Alkyl(meth)acrylaten zugeführt werden.

Vorzugsweise weist die Destillationsapparatur wie geschildert mindestens einen Bereich, Reaktor genannt, auf, in dem mindestens ein Katalysator vorgesehen ist. Dieser Reaktor kann, wie beschrieben, vorzugsweise innerhalb der Destillationskolonne liegen. Es kann von Vorteil sein, wenn höchstens 10 Gew.-%, vorzugsweise höchstens 5 Gew.-% und besonders bevorzugt höchstens 1 Gew.-% des in der Reaktionsphase befindlichen Alkohols über die Gasphase aus dem Reaktionssystem entfernt werden. Durch diese Maßnahme kann die Reaktion besonders kostengünstig durchgeführt werden.

Diese Reaktion kann beispielsweise durch basische Katalysatoren beschleunigt werden. Diese umfassen homogene Katalysatoren sowie heterogene Katalysatoren.

Zu den homogenen Katalysatoren gehören Alkalimetallalkoholate und organometallische Verbindungen von Titan, Zinn und Aluminium. Vorzugsweise wird ein Titanalkoholat oder Zinnalkoholat, wie beispielsweise Titantetraisopropyloxid oder Zinntetrabutyloxid eingesetzt. Zu den heterogenen Katalysatoren gehören u.a. Magnesiumoxid, Calciumoxid sowie basische Ionenaustauscher, wie sie zuvor beschrieben wurden.

Von ganz besonderem Interesse für die Durchführung des Verfahrens sind als Katalysatoren wasserbeständige Lanthanoidverbindungen. Der Einsatz dieser Sorte der homogenen Katalysatoren führt zu überraschend vorteilhaften Ergebnissen. Der Ausdruck "wasserbeständig" bedeutet, dass der Katalysator in Gegenwart von Wasser seine katalytischen Fähigkeiten beibehält. Dementsprechend kann die Umsetzung in Gegenwart von bis zu 2 Gew.-% Wasser erfolgen, ohne dass die katalytische Fähigkeit des Katalysators hierdurch wesentlich beeinträchtigt werden würde. In diesem Zusammenhang bedeutet der Ausdruck "wesentlich", dass die Reaktionsgeschwindigkeit und/oder die Selektivität höchstens um 50%, bezogen auf die Umsetzung ohne Gegenwart von Wasser abnimmt.

Lanthanoidverbindungen bezeichnen Verbindungen von La, Ce, Pr, Nd, Pm, Sm, Eu, Gd, Tb, Dy, Ho, Er, Tm, Yb und/oder Lu. Bevorzugt wird eine Lanthanoidverbindung eingesetzt, die Lanthan umfasst. Vorzugsweise weist die Lanthanoidverbindung eine Löslichkeit in Wasser von mindestens 1 g/l, bevorzugt mindestens 10 g/l bei 25°C auf. Bevorzugte Lanthanoidverbindungen stellen Salze dar, die vorzugsweise in der Oxidationsstufe 3 vorliegen. Besonders bevorzugte wasserbeständige Lanthanoidverbindungen sind La (NO₃)₃ und/oder LaCl₃. Diese Verbindungen können als Salze der Reaktionsmischung zugegeben werden oder in situ gebildet werden.

Eine besondere Verfahrensvariante beinhaltet, dass man als Katalysator einen löslichen Metallkomplex einsetzt, der Titan und/oder Zinn und das alpha-Hydroxycarbonsäureamid enthält.

Eine andere spezielle Abwandlung des Verfahrens sieht vor, dass man als Katalysator ein Metall-trifluormethansulfonat einsetzt. Vorzugsweise setzt man dabei ein Metall-trifluormethansulfonat ein, bei dem das Metall ausgewählt ist aus der Gruppe bestehend aus den Elementen in den Gruppen 1, 2, 3, 4, 11, 12, 13 und 14 des Periodensystems. Hiervon werden bevorzugt solche Metall-trifluormethansulfonate eingesetzt, bei denen das Metall einen oder mehreren Lanthanoiden entspricht.

Neben den bevorzugten Varianten der homogenen Katalyse sind unter Umständen auch Verfahren unter Anwendung heterogener Katalysatoren zweckmäßig. Zu den mit Erfolg anwendbaren heterogenen Katalysatoren gehören unter anderem Magnesiumoxid, Calciumoxid sowie basische Ionenaustauscher und dergleichen mehr. So können beispielsweise Verfahren bevorzugt sein, bei denen der Katalysator ein unlösliches Metalloxid ist, welches mindestens ein aus der aus Sb, Sc, V, La, Ce, Ti, Zr, Hf, V, Nb, Ta, Cr, Mo, W, Tc, Re, Fe, Co, Ni, Cu, Al, Si, Sn, Pb und Bi bestehenden Gruppe ausgewähltes Element enthält. Alternativ dazu können Verfahren bevorzugt sein, wobei man als Katalysator ein unlösliches Metall einsetzt, ausgewählt aus der Gruppe bestehend aus Ti, Zr, Hf, V, Nb, Ta, Cr, Mo, W, Fe, Co, Ni , Cu, Ga, In, Bi und Te. Üblicherweise wird der entstehende Ammoniak aus dem Reaktionssystem abgeleitet, wobei die Umsetzung vielfach beim Siedepunkt durchgeführt wird.

Der bei der Alkoholyse freigesetzte Ammoniak kann dem Gesamtprozess auf leichte Weise zurückgeführt werden. Beispielsweise kann Ammoniak mit Methanol zu Blausäure umgesetzt werden. Dies ist beispielsweise in EP-A-0941984 dargelegt. Des Weiteren kann die Blausäure aus Ammoniak und Methan gemäß dem BMA- oder Andrussow-Verfahren erhalten werden, wobei diese Verfahren in Ullmann's Encyclopedia of Industrial Chemistry 5. Auflage auf CD-ROM, Stichwort "Inorganic Cyano Compounds" beschrieben sind.

Bevorzugte Ausgestaltungen der Alkoholyse des α-Hydroxycarbonsäureamids gemäß Schritt C) werden in WO2007/131829 beschrieben. In einem nächsten Schritt D) wird der α-Hydroxycarbonsäurealkylester mit (Meth)acrylsäure umgesetzt, wobei Alkyl(meth)acrylat sowie α-Hydroxycarbonsäure erhalten werden.

α-Hydroxycarbonsäurealkylester können mit (Meth)acrylsäure umgesetzt werden. Die hierfür einsetzbaren (Meth)acrylsäuren sind an sich bekannt und können kommerziell erhalten werden. Neben Acrylsäure (Propensäure) und Methacrylsäure (2-Methylpropensäure) gehören hierzu insbesondere Derivate, die Substituenten umfassen. Zu den geeigneten Substituenten gehören insbesondere Halogene, wie Chlor, Fluor und Brom sowie Alkylgruppen, die vorzugsweise 1 bis 10, besonders bevorzugt 1 bis 4 Kohlenstoffatome umfassen können. Hierzu gehören unter anderem β-Methylacrylsäure (Butensäure), α, β-Dimethylacrylsäure, β-Ethylacrylsäure, sowie β,β-Dimethylacrylsäure. Bevorzugt sind Acrylsäure (Propensäure) und Methacrylsäure (2-Methylpropensäure), wobei Methacrylsäure besonders bevorzugt ist.

Die hierfür eingesetzten α-Hydroxycarbonsäurealkylester sind an sich bekannt, wobei der Alkoholrest des Esters vorzugsweise 1 bis 20 Kohlenstoffatome, insbesondere 1 bis 10 Kohlenstoffatome und besonders bevorzugt 1 bis 5 Kohlenstoffatome umfasst. Bevorzugte Alkoholreste leiten sich insbesondere von Methanol, Ethanol, Propanol, Butanol, insbesondere n-Butanol und 2-Methyl-1-propanol, Pentanol, Hexanol und 2-Ethylhexanol ab, wobei Methanol und Ethanol besonders bevorzugt sind.

Der Säurerest der zur Umesterung eingesetzten α-Hydroxycarbonsäurealkylester leitet sich bevorzugt von der (Meth)acrylsäure ab, die durch Dehydratisierung der α-Hydroxycarbonsäure erhalten werden kann. Wird beispielsweise Methacrylsäure eingesetzt, so wird α-Hydroxyisobuttersäureester verwendet. Wird beispielsweise Acrylsäure eingesetzt, so wird bevorzugt α-Hydroxyisopropionsäure eingesetzt.

Bevorzugt eingesetzte α-Hydroxycarbonsäurealkylester sind α-Hydroxypropionsäuremethylester, α-Hydroxypropionsäureethylester, α-Hydroxyisobuttersäuremethylester und α-Hydroxyisobuttersäureethylester.

Die Reaktionsmischung kann neben den Reaktanden weitere Bestandteile, wie beispielsweise Lösungsmittel, Katalysatoren, Polymerisationsinhibitoren und Wasser umfassen.

Die Umsetzung des Alkylhydroxycarbonsäureesters mit (Meth)acrylsäure kann durch mindestens eine Säure oder mindestens eine Base katalysiert werden. Hierbei können sowohl homogene als auch heterogene Katalysatoren verwendet werden. Als saure Katalysatoren besonders geeignet sind insbesondere anorganische Säuren, beispielsweise Schwefelsäure oder Salzsäure, sowie organische Säuren, beispielsweise Sulfonsäuren, insbesondere p-Toluolsulfonsäure sowie saure Kationenaustauscher.

Zu den besonders geeigneten Kationenaustauscherharzen gehören insbesondere sulfonsäuregruppenhaltige Styrol-Divinylbenzolpolymere. Besonders geeignete Kationenaustauscherharze können kommerziell von Rohm&Haas unter der Handelsbezeichnung Amberlyst® und von Lanxess unter der Handelsbezeichnung Lewatit® erhalten werden.

Die Konzentration an Katalysator liegt vorzugsweise im Bereich von 1 bis 30 Gew.-%, besonders bevorzugt 5 bis 15 Gew.-%, bezogen auf die Summe des eingesetzten α-Alkylhydroxycarbonsäureesters und der eingesetzten (Meth)acrylsäure.

Zu den bevorzugt einsetzbaren Polymerisationsinhibitoren gehören unter anderem Phenothiazin, Tertiärbutylcatechol, Hydrochinonmonomethylether, Hydrochinon, 4-Hydroxy-2,2,6,6-tetramethylpiperidinooxyl (TEMPOL) oder deren Gemische; wobei die Wirksamkeit dieser Inhibitoren durch Einsatz von Sauerstoff teilweise verbessert werden kann. Die Polymerisationsinhibitoren können in einer Konzentration im Bereich von 0,001 bis 2,0 Gew.-%, besonders bevorzugt im Bereich von 0,01 bis 0,2 Gew.-%, bezogen auf die Summe des eingesetzten α-Alkylhydroxycarbonsäureesters und der eingesetzten (Meth)acrylsäure, eingesetzt werden.

Die Reaktion wird bevorzugt bei Temperaturen im Bereich von 50°C bis 200°C, besonders bevorzugt 70°C bis 130°C, insbesondere 80°C bis 120°C und ganz besonders bevorzugt 90°C bis 110°C durchgeführt.

Die Reaktion kann bei Unter- oder Überdruck durchgeführt werden, je nach Reaktionstemperatur. Vorzugsweise wird diese Reaktion beim Druckbereich von 0,02-5 bar, insbesondere 0,2 bis 3 bar und besonders bevorzugt 0,3 bis 0,5 bar durchgeführt.

Das molare Verhältnis von (Meth)acrylsäure zum α-Hydroxycarbonsäurealkylester liegt vorzugsweise im Bereich von 4:1-1:4, insbesondere 3:1 bis 1:3 und besonders bevorzugt im Bereich von 2:1-1:2.

Bevorzugt beträgt die Selektivität mindestens 90 %, besonders bevorzugt 98 %. Die Selektivität ist definiert als das Verhältnis der Summe aus gebildeten Stoffmengen an Alkyl(meth)acrylaten und α-Hydroxycarbonsäuren, bezogen auf die Summe der umgesetzten Stoffmengen an α-Hydroxycarbonsäurealkylester und (Meth)acrylsäure.

Gemäß einem besonderen Aspekt kann die Umesterung in Gegenwart von Wasser erfolgen. Vorzugsweise liegt der Wassergehalt im Bereich von 0,1-50 Gew.-%, besonders bevorzugt 0,5-20 Gew.-%, und ganz besonders bevorzugt 1-10 Gew.-%, bezogen auf das Gewicht des eingesetzten α-Hydroxycarbonsäurealkylesters.

Durch den Zusatz von geringen Mengen Wasser kann überraschend die Selektivität der Umsetzung erhöht werden. Trotz Wasserzugabe kann dabei die Bildung von Methanol überraschend gering gehalten werden. Bei einer Wasserkonzentration von 10 bis 15 Gew.-%, bezogen auf das Gewicht des eingesetzten α-Hydroxycarbonsäurealkylesters, bilden sich vorzugsweise weniger als 5 Gew.-% Methanol bei einer Reaktionstemperatur von 120°C und einer Reaktionsdauer bzw. Verweilzeit von 5 bis 180 min.

Die Umesterung kann chargenweise oder kontinuierlich durchgeführt werden, wobei kontinuierliche Verfahren bevorzugt sind. Bei der Umesterung können die Produkte vorzugsweise von den Edukten abgetrennt werden, um das Gleichgewicht der Reaktion zu verschieben.

Die Reaktionsdauer der Umesterung hängt von den eingesetzten Molmassen sowie der Reaktionstemperatur ab, wobei dieser Parameter in weiten Bereichen liegen kann. Bevorzugt liegt die Reaktionszeit der Umesterung des α-Hydroxycarbonsäurealkylesters mit (Meth)acrylsäure im Bereich von 30 Sekunden bis 15 Stunden, besonders bevorzugt 5 Minuten bis 5 Stunden und ganz besonders bevorzugt 15 Minuten bis 3 Stunden.

Bei kontinuierlichen Verfahren beträgt die Verweilzeit vorzugsweise 30 Sekunden bis 15 Stunden, besonders bevorzugt 5 Minuten bis 5 Stunden und ganz besonders bevorzugt 15 Minuten bis 3 Stunden.

Bei der Herstellung von Methylmethacrylat aus α-Hydroxyisobuttersäuremethylester beträgt die Temperatur vorzugsweise 60 bis 130 °C, besonders bevorzugt 80 bis 120°C und ganz besonders bevorzugt 90 bis 110 °C. Der Druck liegt vorzugsweise im Bereich von 50 bis 1000 mbar, besonders bevorzugt 300 bis 800 mbar. Das molare Verhältnis von Methacrylsäure zu α-Hydroxyisobuttersäuremethylester liegt vorzugsweise im Bereich von 2:1-1:2, insbesondere 1,5:1-1:1,5.

Gemäß einer besonders bevorzugten Ausführungsform kann die Umesterung in einer Destille erfolgen. Hierbei kann der Katalysator in jedem Bereich der Destille beigefügt werden. Beispielsweise kann der Katalysator in den Bereich des Sumpfes oder im Bereich der Kolonne bereitgestellt werden. Hierbei sollten die Edukte jedoch in Kontakt mit dem Katalysator gebracht werden. Des Weiteren kann Katalysator in einem separaten Bereich der Destille bereitgestellt werden, wobei dieser Bereich mit den weiteren Bereichen der Destille, beispielsweise des Sumpfes und/oder der Kolonne verbunden sind. Diese separate Anordnung des Katalysatorbereichs ist bevorzugt.

Durch diese bevorzugte Ausgestaltung gelingt es überraschend die Selektivität der Umsetzung zu erhöhen. In diesem Zusammenhang ist festzuhalten, dass der Druck der Umsetzung unabhängig vom Druck innerhalb der Destillationskolonnen eingestellt werden kann. Hierdurch kann die Siedetemperatur niedrig gehalten werden, ohne dass die Reaktionszeit bzw. die Verweilzeit entsprechend ansteigt. Darüber hinaus kann die Temperatur der Umsetzung über einen weiten Bereich variiert werden. Hierdurch kann die Reaktionszeit verkürzt werden. Darüber hinaus kann das Volumen an Katalysator beliebig gewählt werden, ohne dass auf die Geometrie der Kolonne Rücksicht genommen werden müsste. Weiterhin kann beispielsweise ein weiterer Reaktand hinzugefügt werden. All diese Maßnahmen können zur Steigerung der Selektivität und der Produktivität beitragen, wobei überraschende Synergieeffekte erzielt werden.

Hierbei wird der α-Hydroxycarbonsäurealkylester, beispielsweise α-Hydroxyisobuttersäuremethylester, der Destille zugeführt. Des Weiteren wird (Meth)acrylsäure, beispielsweise Methacrylsäure in die Destille eingeleitet. Die Destillationsbedingungen werden bevorzugt so ausgeführt, dass genau ein Produkt durch Destillation aus der Destille abgeleitet wird, wobei das zweite Produkt im Sumpf verbleibt und aus diesem kontinuierlich entfernt wird. Bei Verwendung von Alkoholen mit einer geringen Kohlenstoffanzahl, insbesondere Ethanol oder Methanol, wird vorzugsweise das Alkyl(meth)acrylat durch Destillation der Reaktionsmischung entzogen. Die Edukte werden zyklisch durch den Katalysatorbereich geleitet. Hierdurch entsteht kontinuierlich Alkyl(meth)acrylat sowie α-Hydroxycarbonsäure.

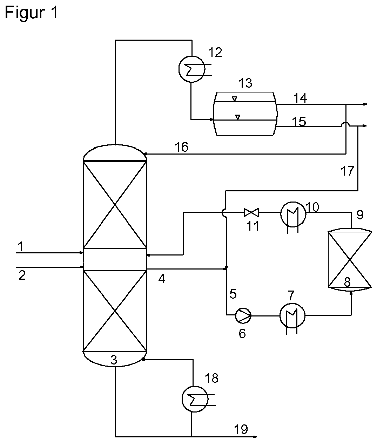

Eine bevorzugte Ausführungsform einer Destille ist in Fig. 1 schematisch dargelegt. Die Edukte können über eine gemeinsame Leitung (1) oder getrennt über zwei Leitungen (1) und (2) in die Destillationskolonne (3) eingeleitet werden. Bevorzugt erfolgt die Zugabe der Edukte über getrennte Leitungen. Die Edukte können dabei auf derselben Stufe oder in beliebiger Position der Kolonne zugeführt werden.

Die Temperatur der Reaktanden kann dabei über einen Wärmetauscher in der Zuführung eingestellt werden, wobei die hierfür notwendigen Aggregate nicht in Figur 1 dargestellt sind. In einer bevorzugten Variante werden die Edukte separat in die Kolonne dosiert, wobei die Zudosierung der leichtersiedenden Komponente unterhalb der Position für die Zuführung der schwersiedenden Verbindung erfolgt. Bevorzugt wird in diesem Fall die leichtersiedende Komponente dampfförmig zugegeben.

Für das Verfahren kann jede mehrstufige Destillationskolonne (3) verwendet werden, die zwei oder mehr Trennstufen besitzt. Als Anzahl der Trennstufen wird in der vorliegenden Erfindung die Anzahl der Böden bei einer Bodenkolonne oder die Anzahl der theoretischen Trennstufen im Fall einer Packungskolonne oder eine Kolonne mit Füllkörpern bezeichnet.

Beispiele für eine mehrstufige Destillationskolonne mit Böden beinhalten solche wie Glockenböden, Siebböden, Tunnelböden, Ventilböden, Schlitzböden, Sieb-Schlitzböden, Sieb-Glockenböden, Düsenböden, Zentrifugalböden, für eine mehrstufige Destillationskolonne mit Füllkörpern solche wie Raschig-Ringe, Lessing-Ringe, Pall-Ringe, Berl-Sättel, Intalox Sättel und für eine mehrstufige Destillationskolonne mit Packungen wie solche vom Typ Mellapak (Sulzer), Rombopak (Kühni), Montz-Pak (Montz) und Packungen mit Katalysatortaschen, beispielsweise Kata-Pak.

Eine Destillationskolonne mit Kombinationen aus Bereichen von Böden, aus Bereichen von Füllkörpern oder aus Bereichen von Packungen kann ebenso verwendet werden.

Die Kolonne (3) kann mit Einbauten ausgestattet sein. Die Kolonne weist vorzugsweise einen Kondensator (12) zur Kondensation des Dampfes und einen Sumpf-Verdampfer (18) auf.

Vorzugsweise weist die Destillationsapparatur mindestens einen Bereich, nachfolgend Reaktor genannt, auf, in dem mindestens ein Katalysator vorgesehen ist. Dieser Reaktor kann innerhalb der Destillationskolonne liegen. Vorzugsweise wird dieser Reaktor jedoch außerhalb der Kolonne (3) in einem separaten Bereich angeordnet, wobei eine dieser bevorzugten Ausführungsformen in Figur 1 näher erläutert wird.

Um die Umesterungsreaktion in einem separaten Reaktor (8) durchzuführen, kann innerhalb der Kolonne ein Teil der abwärts strömenden flüssigen Phase über einen Sammler aufgefangen und als Teilstrom (4) aus der Kolonne geleitet. Die Position des Sammlers wird durch das Konzentrationsprofil in der Kolonne der einzelnen Komponenten bestimmt. Das Konzentrationsprofil kann hierbei über die Temperatur und/oder den Rücklauf geregelt werden. Der Sammler wird bevorzugt so positioniert, dass der aus der Kolonne herausgeführte Strom beide Reaktanden, besonders bevorzugt die Reaktanden in ausreichend hoher Konzentration und ganz besonders bevorzugt im molaren Verhältnis Säure: Ester = 1,5:1 bis 1:1,5 enthält. Des Weiteren können mehrere Sammler an verschiedenen Stellen der Destillationskolonne vorgesehen sein, wobei durch die Menge an entnommenen Reaktanden die molaren Verhältnisse eingestellt werden können.

Dem aus der Kolonne abgeführten Strom kann zudem noch ein weiterer Reaktand, beispielsweise Wasser zudosiert werden, um das Produktverhältnis Säure/Ester in der Kreuzumesterungsreaktion einzustellen oder die Selektivität zu erhöhen. Das Wasser kann über eine Leitung von außen zugeführt (in Figur 1 nicht dargestellt) oder aus einem Phasentrenner (13) entnommen werden. Der Druck des mit Wasser angereicherten Stroms (5) kann anschließend über ein Mittel zur Druckerhöhung (6), beispielsweise eine Pumpe, erhöht werden.

Durch eine Erhöhung des Drucks kann eine Bildung von Dampf in dem Reaktor, beispielsweise einem Festbettreaktor vermindert bzw. verhindert werden. Hierdurch kann eine gleichmäßige Durchströmung des Reaktors und Benetzung der Katalysatorpartikel erzielt werden. Der Strom kann durch einen Wärmetauscher (7) geführt und die Reaktionstemperatur eingestellt werden. Der Strom kann dabei je nach Bedarf erwärmt oder gekühlt werden. Über die Reaktionstemperatur kann zudem das Produktverhältnis Ester zu Säure eingestellt werden.

Im Festbettreaktor (8) findet am Katalysator die Umesterungsreaktion statt. Der Reaktor kann dabei abwärts oder aufwärts durchströmt werden. Der die zu einem gewissen Anteil die Produkte und die nicht umgesetzten Edukte enthaltende Reaktorabstrom (9), wobei der Anteil der Komponenten im Reaktorabstrom von der Verweilzeit, der Katalysatormasse, der Reaktionstemperatur und dem Eduktverhältnis sowie der zugesetzten Wassermenge abhängt, wird zunächst durch einen Wärmetauscher (10) geleitet und auf eine Temperatur eingestellt, die beim Einleiten in die Destillationskolonne vorteilhaft ist. Bevorzugt wird die Temperatur eingestellt, die der Temperatur in der Destillationskolonne an der Stelle des Einleitens des Stroms entspricht.

Die Position, wo der den Reaktor verlassende Strom in die Kolonne zurückgeführt wird, kann dabei ober- oder unterhalb der Position für die Entnahme der Reaktorzuführung liegen, bevorzugt wird jedoch oberhalb. Vor dem Rückführen in die Kolonne kann der Strom über ein Ventil (11) entspannt werden, wobei vorzugsweise das gleiche Druckniveau wie in der Kolonne eingestellt wird. Bevorzugt weist die Destillationskolonne hierbei einen niedrigeren Druck auf. Diese Ausgestaltung bietet den Vorteil, dass die Siedepunkte der zu trennenden Komponenten herabgesetzt werden, wodurch die Destillation auf niedrigerem Temperaturniveau, dadurch energiesparender und thermisch schonender durchgeführt werden kann.

In der Destillationskolonne (3) erfolgt dann die Auftrennung des Produktgemisches. Der Niedersieder, bevorzugt der in der Umesterung gebildete Ester, wird über Kopf abgetrennt. Bevorzugt wird die Destillationskolonne so gefahren, dass das vor dem Festbettreaktor zugegebene Wasser ebenfalls als Kopfprodukt abgetrennt wird. Der am Kopf abgezogene, dampfförmige Strom wird in einem Kondensator (12) kondensiert und anschließend in einem Dekanter (13) in die wässrige und Produktesterhaltige Phase aufgetrennt. Die wässrige Phase kann über die Leitung (15) zur Aufarbeitung ausgeschleust oder vollständig oder teilweise über Leitung (17) wieder in die Reaktion zurückgeführt werden. Der Strom aus der esterhaltigen Phase kann über Leitung (14) zum Teil als Rücklauf (16) auf die Kolonne gefahren werden oder zum Teil aus der Destille ausgeschleust werden. Der Schwersieder, bevorzugt die in der Kreuzumesterung gebildete Säure, wird als Sumpfstrom aus der Kolonne (19) ausgeschleust.

Die aus der Reaktion erhaltene α-Hydroxycarbonsäure, beispielsweise Hydroxyisobuttersäure, kann auf bekannte Weise in einem weiteren Schritt E) dehydratisiert werden. Im Allgemeinen wird die α-Hydroxycarbonsäure, beispielsweise die α-Hydroxyisobuttersäure in Gegenwart mindestens eines Metallsalzes, beispielsweise von Alkali- und/oder Erdalkalimetallsalzen, auf Temperaturen im Bereich von 160-300°C, besonders bevorzugt im Bereich von 200 bis 240 °C erhitzt, wobei im Allgemeinen die (Meth)acrylsäure sowie Wasser erhalten werden. Zu den geeigneten Metallsalzen gehören u.a. Natriumhydroxid, Kaliumhydroxid, Calciumhydroxid, Bariumhydroxid, Magnesiumhydroxid, Natriumsulfit, Natriumcarbonat, Kaliumcarbonat, Strontiumcarbonat, Magnesiumcarbonat, Natriumbicarbonat, Natriumacetat, Kaliumacetat und Natriumdihydrogenphosphat.

Die Dehydratisierung der α-Hydroxycarbonsäure kann vorzugsweise bei einem Druck im Bereich von 0,05 bar bis 2,5 bar, besonders bevorzugt im Bereich von 0,1 bar bis 1 bar durchgeführt werden.

Die Dehydratisierung von α-Hydroxycarbonsäuren ist beispielsweise in DE-A-176 82 53 beschrieben.

Die so erhaltene (Meth)acrylsäure kann wiederum zur Herstellung von Alkyl(meth)acrylaten eingesetzt werden. Des Weiteren ist (Meth)acrylsäure ein Handelsprodukt. Überraschend kann dementsprechend das Verfahren zur Herstellung von Alkyl(meth)acrylaten ebenfalls dazu dienen, (Meth)acrylsäure herzustellen, wobei das Produktverhältnis von Alkyl(meth)acrylaten zu (Meth)acrylsäure durch die Konzentration an Wasser bei der Umesterung des α-Hydroxycarbonsäurealkylesters und/oder durch die Reaktionstemperatur leicht reguliert werden kann.

Das erfindungsgemäße Verfahren kann als Teilschritt eines Verfahrens zur Herstellung von Polymeren, Formmassen und Kunststoffformkörpern angesehen werden. Das erfindungsgemäß herzustellende Methylmethacrylat, kann radikalisch zu Polymeren umgesetzt werden.

Diese Polymere werden im Allgemeinen durch radikalische Polymerisation von Mischungen erhalten, die Methylmethacrylat enthalten. Im Allgemeinen enthalten diese Mischungen mindestens 40 Gew.-%, vorzugsweise mindestens 60 Gew.-% und besonders bevorzugt mindestens 80 Gew.-%, bezogen auf das Gewicht der Monomere, Methylmethacrylat.

Daneben können diese Mischungen weitere (Meth)acrylate enthalten, die mit Methylmethacrylat copolymerisierbar sind. Der Ausdruck (Meth)acrylate umfasst Methacrylate und Acrylate sowie Mischungen aus beiden.

Diese Monomere sind weithin bekannt. Zu diesen gehören unter anderem (Meth)acrylate, die sich von gesättigten Alkoholen ableiten, wie beispielsweise Methylacrylat, Ethyl(meth)acrylat, Propyl(meth)acrylat, n-Butyl(meth)acrylat, tert.-Butyl(meth)acrylat, Pentyl(meth)acrylat und 2-Ethylhexyl(meth)acrylat; (Meth)acrylate, die sich von ungesättigten Alkoholen ableiten, wie z. B. O-leyl(meth)acrylat, 2-Propinyl(meth)acrylat, Allyl(meth)acrylat, Vinyl(meth)acrylat; Aryl(meth)acrylate, wie Benzyl(meth)acrylat oder Phenyl(meth)acrylat, wobei die Arylreste jeweils unsubstituiert oder bis zu vierfach substituiert sein können;
Cycloalkyl(meth)acrylate, wie 3-Vinylcyclohexyl(meth)acrylat, Bornyl(meth)acrylat; Hydroxylalkyl(meth)acrylate, wie
3-Hydroxypropyl(meth)acrylat,
3,4-Dihydroxybutyl(meth)acrylat,
2-Hydroxyethyl(meth)acrylat, 2-Hydroxypropyl(meth)acrylat; Glycoldi(meth)acrylate, wie 1,4-Butandiol(meth)acrylat,
(Meth)acrylate von Etheralkoholen, wie
Tetrahydrofurfuryl(meth)acrylat, Vinyloxyethoxyethyl(meth)acrylat;
Amide und Nitrile der (Meth)acrylsäure, wie
N-(3-Dimethylaminopropyl)(meth)acrylamid,
N-(Diethylphosphono)(meth)acrylamid,
1-Methacryloylamido-2-methyl-2-propanol;
schwefelhaltige Methacrylate, wie
Ethylsulfinylethyl(meth)acrylat,
4-Thiocyanatobutyl(meth)acrylat,
Ethylsulfonylethyl(meth)acrylat,
Thiocyanatomethyl(meth)acrylat,
Methylsulfinylmethyl(meth)acrylat,
Bis((meth)acryloyloxyethyl)sulfid;
mehrwertige (Meth)acrylate, wie
Trimethyloylpropantri(meth)acrylat.

Neben den zuvor dargelegten (Meth)acrylaten können die zu polymerisierenden Zusammensetzungen auch weitere ungesättigte Monomere aufweisen, die mit Methylmethacrylat und den zuvor genannten (Meth)acrylaten copolymerisierbar sind. Hierzu gehören unter anderem 1-Alkene, wie Hexen-1, Hepten-1; verzweigte Alkene, wie beispielsweise Vinylcyclohexan, 3,3-Dimethyl-1-propen, 3-Methyl-1-diisobutylen, 4-Methylpenten-1;
Acrylnitril; Vinylester, wie Vinylacetat;
Styrol, substituierte Styrole mit einem Alkylsubstituenten in der Seitenkette, wie z. B. α -Methylstyrol und α-Ethylstyrol, substituierte Styrole mit einem Alkylsubstitutenten am Ring, wie Vinyltoluol und p-Methylstyrol, halogenierte Styrole, wie beispielsweise Monochlorstyrole, Dichlorstyrole, Tribromstyrole und Tetrabromstyrole;
Heterocyclische Vinylverbindungen, wie 2-Vinylpyridin, 3-Vinylpyridin, 2-Methyl-5-vinylpyridin, 3-Ethyl-4-vinylpyridin, 2,3-Dimethyl-5-vinylpyridin, Vinylpyrimidin, Vinylpiperidin, 9-Vinylcarbazol, 3-Vinylcarbazol, 4-Vinylcarbazol, 1-Vinylimidazol, 2-Methyl-1-vinylimidazol, N-Vinylpyrrolidon, 2-Vinylpyrrolidon, N-Vinylpyrrolidin, 3-Vinylpyrrolidin, N-Vinylcaprolactam, N-Vinylbutyrolactam, Vinyloxolan, Vinylfuran, Vinylthiophen, Vinylthiolan, Vinylthiazole und hydrierte Vinylthiazole, Vinyloxazole und hydrierte Vinyloxazole;
Vinyl- und Isoprenylether;
Maleinsäurederivate, wie beispielsweise Maleinsäureanhydrid, Methylmaleinsäureanhydrid, Maleinimid, Methylmaleinimid; und
Diene, wie beispielsweise Divinylbenzol.

Im allgemeinen werden diese Comonomere in einer Menge von 0 bis 60 Gew.-%, vorzugsweise 0 bis 40 Gew.-% und besonders bevorzugt 0 bis 20 Gew.-%, bezogen auf das Gewicht der Monomeren, eingesetzt, wobei die Verbindungen einzeln oder als Mischung verwendet werden können.

Die Polymerisation wird im Allgemeinen mit bekannten Radikalinitiatoren gestartet. Zu den bevorzugten Initiatoren gehören unter anderem die in der Fachwelt weithin bekannten Azoinitiatoren, wie AIBN und 1,1-Azobiscyclohexancarbonitril, sowie Peroxyverbindungen, wie Methylethylketonperoxid, Acetylacetonperoxid, Dilaurylperoxyd, tert.-Butylper-2-ethylhexanoat, Ketonperoxid, Methylisobutylketonperoxid, Cyclohexanonperoxid, Dibenzoylperoxid, tert.-Butylperoxybenzoat, tert.-Butylperoxyisopropylcarbonat, 2,5-Bis(2-ethylhexanoyl-peroxy)-2,5-dimethylhexan, tert.-Butylperoxy-2-ethylhexanoat, tert.-Butylperoxy-3,5,5-trimethylhexanoat, Dicumylperoxid, 1,1-Bis(tert.-butylperoxy)cyclohexan, 1,1-Bis(tert.-butylperoxy)3,3,5-trimethylcyclohexan, Cumylhydroperoxid, tert.-Butylhydroperoxid, Bis(4-tert.-butylcyclohexyl)peroxydicarbonat, Mischungen von zwei oder mehr der vorgenannten Verbindungen miteinander sowie Mischungen der vorgenannten Verbindungen mit nicht genannten Verbindungen, die ebenfalls Radikale bilden können.

Diese Verbindungen werden häufig in einer Menge von 0,01 bis 10 Gew.-%, vorzugsweise von 0,5 bis 3 Gew.-%, bezogen auf das Gewicht der Monomeren, eingesetzt.

Vorzugsweise kann die Polymerisation bei einer Temperatur im Bereich von 20°C bis 120°C durchgeführt werden.

Die Herstellung der (Meth)acrylat-Homo- und/oder Copolymere aus (Meth)acrylaten nach den verschiedenen Verfahren der radikalischen Polymerisation ist an sich bekannt. So können die Polymere in Substanz-, Lösungs-, Suspensions- oder Emulsionspolymerisation hergestellt werden. Die Substanzpolymerisation ist beispielhaft in Houben-Weyl, Band E20, Teil 2 (1987), S. 1145ff. beschrieben. Wertvolle Hinweise hinsichtlich der Lösungspolymerisation finden sich eben dort auf S. 1156ff. Erläuterungen zur Suspensionspolymerisationstechnik finden sich eben dort auf S. 1149ff, während die Emulsionspolymerisation eben dort auf S. 1150ff. ausgeführt und erläutert wird.

Die zuvor dargelegten Polymere können insbesondere zur Herstellung von Formmassen eingesetzt werden, die üblich neben den Polymeren Additive umfassen können, wie zum Beispiel Farbmittel, Pigmente, beispielsweise Metallic-Pigmente, UV-Stabilisatoren oder Füllstoffe. Der Anteil dieser Additive ist von der beabsichtigten Anwendung abhängig und kann daher in weiten Bereichen liegen. Vorzugsweise kann dieser Anteil, falls Additive enthalten sind, 0 bis 30 Gew.-%, besonders bevorzugt 0,1 bis 5 Gew.-% betragen.

Die bevorzugten Formmassen bzw. Polymere können über übliche Formgebungsverfahren, beispielsweise Spritzguss oder Extrusion zu Formkörpern verarbeitet werden. Weiterhin kann Methylacrylat, welches über das erfindungsgemäße Verfahren erhalten wurde, zur Herstellung von Gussgläsern eingesetzt werden. Diese gemäß dem Gusskammerverfahren erzeugten Polymere weisen ein besonders hohes Molekulargewicht auf und zeigen daher gegenüber den thermoplastisch verarbeitbaren Polymeren andere mechanische Eigenschaften.

Nachfolgend soll die vorliegende Erfindung anhand eines Beispiels näher erläutert werden.

### Referenzbeispiel 1

Eine Mischung umfassend 1 mol HCN, 2,5 mol Aceton und 200 ppm Li₂O wurde hergestellt und bis zur Einstellung des Gleichgewichts bei 20°C umgesetzt. Die erhaltene Reaktionsmischung umfasste 47,7 Gew.-% Acetoncyanhydrin und 51,5 Gew.-% Aceton. Der Anteil an HCN betrug weniger als 8000 ppm.

Eine Mischung dieser Zusammensetzung wurde kontinuierlich in einen Hydrolysereaktor überführt, in den 38,6 Gew.-% Wasser zugegeben wurden. Der Anteil an HCN blieb hierbei im Wesentlichen unverändert.

Das Wasser wurde teilweise aus einem Rücklauf zugegeben, der bei einer Aufreinigung der Reaktionsmischung nach der Hydrolyse des Acetoncyanhydrin aus dem Sumpf gewonnen wurde. Die Hydrolyse wurde unter Verwendung eines MnO₂-Katalysators durchgeführt, der insbesondere in den Beispielen der WO 2008/061822 näher dargelegt ist. Zur Stabilisierung des Katalysators wurde Luft eingesetzt, wie dies in Beispiel 1 der Druckschrift WO 2008/061822 beschrieben ist. Der pH-Wert betrug 9,0.

Aus der nach der Hydrolyse erhaltenen Reaktionsmischung wurde in einem ersten Destillationsschritt das Carbonsäureamid über den Sumpf abgetrennt, wobei diese Destillation bei einer Temperatur von 175 °C (Sumpftemperatur) und einem Druck von 0,4 bar durchgeführt wurde.

Das Kopfprodukt umfassend 52 Gew.-% Wasser, 43 Gew.-% Aceton, 4,8 Gew.-% Acetoncyanhydrin und 0,2 Gew.-% HCN wurde über eine Destillationskolonne mit Einbauten kontinuierlich aufgereinigt, wobei ein Strom von 1,2 kg/h zugegeben wurde. Über den Rücklauf der Kolonne wurde 0,3 kg Aceton zugeführt, so dass eine hohe Trennleistung erzielt wurde. Die Destillationsanlage hatte eine unbegrenzte Standzeit, d.h. innerhalb von 60 Tagen wurde keine Verringerung der Trennleistung festgestellt. Im Sumpfprodukt konnte kein HCN nachgewiesen werden. Das Kopfprodukt umfasste 86 Gew.-% Aceton, 5,8 Gew.-% Acetoncyanhydrin, 0,2 Gew.-% HCN und 8 Gew.-% Wasser.

Die Standzeit des Katalysators betrug mehr als 60 Tage, wobei die Standzeit des Katalysators definiert ist als Dauer bis zu der der Umsatzgrad unter 50% des Anfangsumsatzgrades fällt.

## Patentansprüche

1. Verfahren zur Herstellung von einem Carbonsäureamid aus einer Carbonylverbindung und Blausäure, umfassend die Schritte
A) Umsetzung einer Carbonylverbindung mit Blausäure zur Herstellung eines Hydroxycarbonsäurenitrils,
B) Hydrolyse des in Schritt A) erhaltenen Hydroxycarbonsäurenitrils in Gegenwart eines Mangandioxid umfassenden Katalysators, **dadurch gekennzeichnet, dass** zur Umsetzung der Carbonylverbindung mit Blausäure gemäß Schritt A) ein molarer Überschuss an Carbonylverbindung in Bezug auf die Blausäure eingesetzt wird und die in Schritt A) erhaltene Reaktionsmischung nicht durch Destillation aufgereinigt wird, bevor die Hydrolyse gemäß Schritt B) durchgeführt wird,
dass das molare Verhältnis von Carbonylverbindung zu Blausäure im Bereich von 2:1 bis 7:1 liegt,
die nach Schritt B) erhaltene Reaktionsmischung durch eine zweistufige Destillation aufgereinigt wird,
das erhaltene Carbonsäureamid und Wasser von einer Mischung getrennt wird, die Carbonylverbindung und Hydroxycarbonsäurenitril und/oder Blausäure umfasst, und in einem weiteren Destillationsschritt Wasser von erhaltenem Carbonsäureamid getrennt wird,
über den Rücklauf in zur Trennung von Wasser und Blausäure eingesetzte Destille (erster Destillationsschritt) eine Zusammensetzung eingeleitet wird, die die in Schritt A)
eingesetzte Carbonylverbindung umfasst, wobei die in den Rücklauf eingeleitete Zusammensetzung einen geringeren Anteil an HCN aufweist, als die über Kopf der Destille entnommene Zusammensetzung durch Zugabe der Carbonylverbindung,
wobei die in Schritt A) eingesetzte Carbonylverbindung Aceton ist.

2. Verfahren gemäß Anspruch 1, **dadurch gekennzeichnet, dass** das Carbonsäureamid von Wasser in einer mehrstufigen Eindampfung getrennt wird.

3. Verfahren gemäß Anspruch 1, **dadurch gekennzeichnet, dass** die Menge an zugegebener Carbonylverbindung so gewählt wird, dass diese zur Herstellung der in Schritt A) vorgesehenen Menge an Hydroxycarbonsäurenitril ausreicht.

4. Verfahren gemäß mindestens einem der Ansprüche 1 bis 3, **dadurch gekennzeichnet, dass** die durch die Aufreinigung erhaltene Carbonylverbindung zur Herstellung eines Hydroxycarbonsäurenitrils gemäß Schritt A) eingesetzt wird.

5. Verfahren gemäß mindestens einem der vorhergehenden Ansprüche, **dadurch gekennzeichnet, dass** die Umsetzung einer Carbonylverbindung mit Blausäure zur Herstellung eines Hydroxycarbonsäurenitrils gemäß Schritt A) bei einer Temperatur im Bereich von -10 bis 50°C durchgeführt wird.

6. Verfahren gemäß mindestens einem der vorhergehenden Ansprüche, **dadurch gekennzeichnet, dass** die zum Mangandioxid umfassenden Katalysator in Schritt B) gegebene Reaktionsmischung einen pH-Wert im Bereich von 6,0 bis 11,0 aufweist und die Hydrolyse in Gegenwart eines Oxidationsmittels durchgeführt wird.

7. Verfahren gemäß Anspruch 6 **dadurch gekennzeichnet, dass** der pH-Wert durch Zugaben von Hydroxiden oder Oxiden eingestellt wird.

8. Verfahren gemäß Anspruch 7, **dadurch gekennzeichnet, dass** die Zugabe zur Einstellung des pH-Wertes bei der Umsetzung einer Carbonylverbindung mit Blausäure zur Herstellung eines Hydroxycarbonsäurenitrils gemäß Schritt A) vorgenommen wird.

9. Verfahren gemäß mindestens einem der vorhergehenden Ansprüche, **dadurch gekennzeichnet, dass** die Hydrolyse in Gegenwart von Lithiumionen durchgeführt wird.

10. Verfahren zur Herstellung von Methylmethacrylat, **dadurch gekennzeichnet, dass** ein Verfahren zur Herstellung von einem Carbonsäureamid aus einer Carbonylverbindung und Blausäure gemäß mindestens einem der Ansprüche 1 bis 9 durchgeführt wird.

## Claims

1. Process for preparing a carboxamide from a carbonyl compound and hydrogen cyanide, comprising the steps of
A) reacting a carbonyl compound with hydrogen cyanide to prepare a hydroxycarbonitrile,
B) hydrolysing the hydroxycarbonitrile obtained in step A) in the presence of a catalyst comprising manganese dioxide, **characterized in that** a molar excess of carbonyl compound in relation to the hydrogen cyanide is used for the reaction of the carbonyl compound with hydrogen cyanide in step A), and the reaction mixture obtained in step A) is not purified by distillation before the hydrolysis in step B) is performed,
**in that** the molar ratio of carbonyl compound to hydrogen cyanide is in the range from 2:1 to 7:1, the reaction mixture obtained after step B) is purified by a two-stage distillation,
the resulting carboxamide and water are separated from a mixture which comprises carbonyl compound and hydroxycarbonitrile and/or hydrogen cyanide, and water is separated from resulting carboxamide in a further distillation step,
a composition which comprises the carbonyl compound used in step A) is introduced via the reflux into a still used to separate water and hydrogen cyanide (first distillation step), wherein the composition introduced into the reflux has a lower proportion of HCN than the composition withdrawn via the top of the still by virtue of addition of the carbonyl compound,
wherein the carbonyl compound used in step A) is acetone.

2. Process according to Claim 1, **characterized in that** the carboxamide is separated from water in a multistage evaporative concentration.

3. Process according to Claim 1, **characterized in that** the amount of carbonyl compound added is selected such that it is sufficient to prepare the amount of hydroxycarbonitrile envisaged in step A).

4. Process according to at least one of Claims 1 to 3, **characterized in that** the carbonyl compound obtained by the purification is used to prepare a hydroxycarbonitrile in step A).

5. Process according to at least one of the preceding claims, **characterized in that** the reaction of a carbonyl compound with hydrogen cyanide to prepare a hydroxycarbonitrile in step A) is performed at a temperature in the range from -10 to 50°C.

6. Process according to at least one of the preceding claims, **characterized in that** the reaction mixture added to the catalyst comprising manganese dioxide in step B) has a pH in the range from 6.0 to 11.0 and the hydrolysis is performed in the presence of an oxidizing agent.

7. Process according to Claim 6, **characterized in that** the pH is adjusted by additions of hydroxides or oxides.

8. Process according to Claim 7, **characterized in that** the addition to adjust the pH is undertaken in the course of reaction of a carbonyl compound with hydrogen cyanide to prepare a hydroxycarbonitrile in step A).

9. Process according to at least one of the preceding claims, **characterized in that** the hydrolysis is performed in the presence of lithium ions.

10. Process for preparing methyl methacrylate, **characterized in that** a process for preparing a carboxamide from a carbonyl compound and hydrogen cyanide according to at least one of Claims 1 to 9 is performed.

## Revendications

1. Procédé de fabrication d'un amide d'acide carboxylique à partir d'un composé de carbonyle et d'acide cyanhydrique, comprenant les étapes suivantes :
A) la mise en réaction d'un composé de carbonyle avec de l'acide cyanhydrique pour la fabrication d'un nitrile d'acide hydroxycarboxylique,
B) l'hydrolyse du nitrile d'acide hydroxycarboxylique obtenu à l'étape A) en présence d'un catalyseur comprenant du dioxyde de manganèse, **caractérisé en ce que**, pour la réaction du composé de carbonyle avec l'acide cyanhydrique selon l'étape A), un excès molaire de composé de carbonyle par rapport à l'acide cyanhydrique est utilisé et le mélange réactionnel obtenu à l'étape A) n'est pas purifié par distillation avant la réalisation de l'hydrolyse selon l'étape B),
**en ce que** le rapport molaire entre le composé de carbonyle et l'acide cyanhydrique se situe dans la plage allant de 2:1 à 7:1,
le mélange réactionnel obtenu à l'étape B) est purifié par une distillation à deux étapes,
l'amide d'acide carboxylique obtenu et de l'eau sont séparés d'un mélange qui comprend le composé de carbonyle et le nitrile d'acide hydroxycarboxylique et/ou l'acide cyanhydrique et, lors d'une étape de distillation supplémentaire, l'eau est séparée de l'amide d'acide carboxylique obtenu,
une composition est introduite par le reflux dans la distillation utilisée pour la séparation de l'eau et de l'acide cyanhydrique (première étape de distillation), qui comprend le composé de carbonyle utilisé à l'étape A), la composition introduite dans le reflux comprenant une proportion d'HCN plus faible que la composition soutirée par la tête de la distillation par ajout du composé de carbonyle,
le composé de carbonyle utilisé à l'étape A) étant l'acétone.

2. Procédé selon la revendication 1, **caractérisé en ce que** l'amide d'acide carboxylique est séparé de l'eau par une évaporation à plusieurs étapes.

3. Procédé selon la revendication 1, **caractérisé en ce que** la quantité de composé de carbonyle ajoutée est choisie de sorte que celle-ci suffise pour la fabrication de la quantité prévue de nitrile d'acide hydroxycarboxylique à l'étape A).

4. Procédé selon au moins l'une quelconque des revendications 1 à 3, **caractérisé en ce que** le composé de carbonyle obtenu par la purification est utilisé pour la fabrication d'un nitrile d'acide hydroxycarboxylique selon l'étape A).

5. Procédé selon au moins l'une quelconque des revendications précédentes, **caractérisé en ce que** la mise en réaction d'un composé de carbonyle avec de l'acide cyanhydrique pour la fabrication d'un nitrile d'acide hydroxycarboxylique selon l'étape A) est réalisée à une température dans la plage allant de -10 à 50 °C.

6. Procédé selon au moins l'une quelconque des revendications précédentes, **caractérisé en ce que** le mélange réactionnel ajouté au catalyseur comprenant du dioxyde de manganèse à l'étape B) présente un pH dans la plage allant de 6,0 à 11,0 et l'hydrolyse est réalisée en présence d'un oxydant.

7. Procédé selon la revendication 6, **caractérisé en ce que** le pH est ajusté par ajout d'hydroxydes ou d'oxydes.

8. Procédé selon la revendication 7, **caractérisé en ce que** l'ajout pour l'ajustement du pH est réalisé lors de la réaction d'un composé de carbonyle avec de l'acide cyanhydrique pour la fabrication d'un nitrile d'acide hydroxycarboxylique selon l'étape A).

9. Procédé selon au moins l'une quelconque des revendications précédentes, **caractérisé en ce que** l'hydrolyse est réalisée en présence d'ions lithium.

10. Procédé de fabrication de méthacrylate de méthyle, **caractérisé en ce qu'**un procédé de fabrication d'un amide d'acide carboxylique à partir d'un composé de carbonyle et d'acide cyanhydrique selon au moins l'une quelconque des revendications 1 à 9 est réalisé.
